(19) Europäisches Patentamt
European Patent Office
Office européen des brevets

(11) **EP 4 141 103 A1**

(12) **DEMANDE DE BREVET EUROPEEN**

(43) Date de publication:
**01.03.2023 Bulletin 2023/09**

(21) Numéro de dépôt: **22192942.5**

(22) Date de dépôt: **30.08.2022**

(51) Classification Internationale des Brevets (IPC):
*C12N 1/20* (2006.01)    *A01N 63/20* (2020.01)
*A01P 21/00* (2006.01)    *C12R 1/01* (2006.01)

(52) Classification Coopérative des Brevets (CPC):
**C12N 1/205; A01N 63/20; A01P 21/00; C12N 1/20;**
C12R 2001/01

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**KH MA MD TN**

(30) Priorité: **31.08.2021 FR 2109098**

(83) **Déclaration conformément à la règle 32(1) CBE
(solution de l'expert)**

(71) Demandeurs:
• **Agro Industrie Recherches et
Developpements A.R.D.
51110 Pomacle (FR)**
• **Université De Reims Champagne-Ardenne
51100 Reims (FR)**

(72) Inventeurs:
• **CAMAILLE, Manon
10400 NOGENT-SUR SEINE (FR)**
• **CLEMENT, Christophe
ROIZY 08190 (FR)**
• **AIT BARKA, Essaid
51110 AUMENANCOURT (FR)**
• **FABRE, Nicolas
WITRY-LES-REIMS 51420 (FR)**
• **SANHAJI, Ghislain
51110 WARMERIVILLE (FR)**

(74) Mandataire: **Grosset-Fournier, Chantal Catherine
et al
Grosset-Fournier & Demachy
54, rue Saint-Lazare
75009 Paris (FR)**

Remarques:
Le document complet y compris le(s) table(s) de
référence et le(s) listage(s) de séquence sont
téléchargeables sur le site internet de l'OEB

(54) **NOUVEL AGENT DE BIOSTIMULATION ET SES UTILISATIONS**

(57) L'invention concerne l'utilisation d'une souche isolée de *Chitinophage sp.* et s'inscrit plus largement dans le domaine de l'agriculture. En particulier, celle-ci concerne l'utilisation de ladite souche comme agent de biostimulation, par exemple, comme agent de lutte contre le stress hydrique et/ou comme agent biofertilisant. Ce faisant, l'invention concerne des compositions phytostimulantes comprenant ladite souche isolée de *Chitinophage sp.*, des procédés les utilisant ainsi que ladite souche en tant que telle.

EP 4 141 103 A1

**Description**

**DOMAINE DE L'INVENTION**

L'invention concerne l'utilisation d'une souche isolée de *Chitinophage sp.* et s'inscrit plus largement dans le domaine de l'agriculture. En particulier, celle-ci concerne l'utilisation de ladite souche comme agent de biostimulation, par exemple, comme agent de lutte contre le stress hydrique et/ou comme agent biofertilisant. Ce faisant, l'invention concerne des compositions phytostimulantes comprenant ladite souche isolée de *Chitinophage sp.*, des procédés les utilisant ainsi que ladite souche en tant que telle.

**ART ANTERIEUR**

Le changement climatique et l'augmentation de la population humaine conduit l'Homme à faire face à de multiples défis, notamment dans l'agriculture. En effet, la production agricole doit augmenter alors que les conditions de culture tendent à se détériorer. La sécheresse constitue en effet l'une des principales limitations pour le rendement des cultures, si ce n'est le problème majeur. Son impact affecte 1 à 3 % de toutes les terres avec pour conséquence une croissance et une productivité limitées des cultures. Ce phénomène est d'ailleurs amené à s'accentuer du fait que dans les années à venir, la disponibilité de l'eau pour l'agriculture sera également un problème d'envergure.

Pour faire face à des conditions défavorables de déficit hydrique, les plantes utilisent des mécanismes sophistiqués et complexes qui les aident à percevoir le signal de stress et à obtenir un rendement optimal. Parmi ces mécanismes, certains s'appuient sur la présence de rhizobactéries dans sol et le tissu racinaire des plantes. Ce faisant, ces bactéries peuvent permettre une meilleure croissance et un rendement plus élevé grâce à la production de phytohormones, d'osmolytes, d'antioxidants, de composés volatils, d'exopolysaccharides, *etc.* (Camaille M.; Fabre N.; Clément C.; Ait Barka E. Plant Growth Promoting Rhizobacteria: a sustainable wat to trigger drought tolerance in wheat. Microorganisms 2021, 9, x.).

Parmi ces rhizobactéries et de manière non exhaustive, peuvent être citées **Bacillus subtilis** (Lastochkina O. (2019) Bacillus subtilis-Mediated Abiotic Stress Tolerance in Plants. In: Islam M., Rahman M., Pandey P., Boehme M., Haesaert G. (eds) Bacilli and Agrobiotechnology: Phytostimulation and Biocontrol. Bacilli in Climate Resilient Agriculture and Bioprospecting. Springer, Cham.) et **Ensifer adhaerens** (Zhou GC, Wang Y, Zhai S, Ge F, Liu ZH, Dai YJ, Yuan S, Hou JY. Biodegradation of the neonicotinoid insecticide thiamethoxam by the nitrogen-fixing and plant-growth-promoting rhizobacterium Ensifer adhaerens strain TMX-23. Appl Microbiol Biotechnol. 2013 May;97(9):4065-74).

Seulement et bien qu'elles soient décrites comme de bons agents de lutte contre le stress hydrique, *Bacillus subtitis* et *Ensifer adhaerens*, ne peuvent pas à elles seules résoudre l'ensemble des problématiques agricoles. En effet, la diversité des cultures agricoles et celle des territoires agricoles nécessitent que soient diversifiés les outils de lutte contre le stress hydrique.

**BREF APERÇU**

Dans ce contexte d'amélioration des rendements agricoles et de lutte contre le stress hydrique subit par les cultures agricoles, un premier but de l'invention concerne la mise à disposition d'une souche isolée de *Chitinophaga pinensis* et son utilisation comme agent de biostimulation. Un deuxième but vise à fournir des compositions phytostimulantes pour notamment la mise en œuvre de procédé soit pour prévenir et/ou lutter contre le stress hydrique subit par un végétal, soit pour stimuler l'assimilation des minéraux par un végétal. Un autre but de l'invention concerne des semences enrobées où sont associées des graines de végétal et la souche isolée de *Chitinophaga pinensis.*

**DESCRIPTION DETAILLEE**

Selon un premier aspect de l'invention, celle-ci a pour objet l'utilisation d'une souche isolée de *Chitinophaga sp.* en tant qu'agent de biostimulation.

Par « agent de biostimulation » ou biostimulant, on entend des substances et/ou des microorganismes dont la fonction, lorsqu'appliqués aux plantes ou à la rhizosphère, est la stimulation des processus naturels qui favorisent/améliorent :

- l'absorption ou l'utilisation des nutriments/minéraux ;
- la tolérance aux stress abiotiques, dont la tolérance au stress hydrique ; et/ou
- la qualité ou le rendement de la culture, indépendamment de la présence de nutriments.

De manière surprenante, il a été identifié que l'espèce *C. pinensis* lorsqu'elle est utilisée répond parfaitement à cette définition et de fait, constitue un outil de choix qui peut être facilement mis à disposition de l'agriculture. Ce faisant, l'utilisation proposée ci-dessus englobe l'obtention d'effets biofertilisants (amélioration de l'assimilation des minéraux et/ou de la qualité ou du rendement de la culture) et/ou de tolérance aux stress abiotiques (dont le stress hydrique).

Autrement dit, un autre mode de réalisation concerne l'utilisation d'une souche isolée de *Chitinophaga pinensis* en tant qu'agent de biostimulation, en particulier en tant qu'agent de lutte contre le stress hydrique et/ou en tant qu'agent biofertilisant.

Par « agent de lutte contre le stress hydrique », on entend que la souche isolée de *Chitinophaga pinensis* une fois appliquée sur un végétal est capable d'accroître la tolérance du végétal au stress hydrique (manque d'eau). Celle-ci peut d'ailleurs être utilisée soit de manière préventive, *i.e.* en prévision d'un épisode de stress hydrique, soit de manière curative, *i.e.* après l'apparition du stress hydrique et en traitement de celui-ci. Un autre mode de réalisation concerne donc l'utilisation d'une souche isolée de *Chitinophaga pinensis* telle que décrite ci-dessus dans la prévention et/ou le traitement du stress hydrique subi par un végétal, en particulier ledit végétal appartenant à la famille des Poaceae tels que le blé, le riz, le maïs, l'orge, le millet, le seigle, l'épeautre, l'engrain ou petit épeautre, ou le sésame.

En particulier, un autre mode de réalisation concerne l'utilisation d'une souche isolée de *Chitinophaga pinensis* telle que décrite ci-dessus dans le traitement du stress hydrique subi par un végétal, en particulier ledit végétal appartenant à la famille des *Poaceae* tels que le blé, le riz, le maïs, l'orge, le millet, le seigle, l'épeautre, l'engrain ou petit épeautre, ou le sésame.

En particulier, un autre mode de réalisation concerne l'utilisation d'une souche isolée de *Chitinophaga pinensis* telle que décrite ci-dessus dans la prévention du stress hydrique subi par un végétal, en particulier ledit végétal appartenant à la famille des *Poaceae* tels que le blé, le riz, le maïs, l'orge, le millet, le seigle, l'épeautre, l'engrain ou petit épeautre, ou le sésame.

Par « agent biofertilisant », on entend que la souche isolée de *Chitinophaga pinensis* une fois appliquée sur un végétal est capable de promouvoir la croissance et le développement du végétal, notamment en favorisant l'assimilation des minéraux par le végétal. Un autre mode de réalisation concerne donc l'utilisation d'une souche isolée de *Chitinophaga pinensis* telle que décrite ci-dessus pour stimuler l'assimilation des minéraux par un végétal, en particulier ledit végétal appartenant à la famille des *Poaceae* tels que le blé, le riz, le maïs, l'orge, le millet, le seigle, l'épeautre, l'engrain ou petit épeautre, ou le sésame.

Ceci étant, il convient de noter que les rôles d'agent de lutte contre le stress hydrique et d'agent biofertilisant ne sont pas antinomiques et peuvent se cumuler. En effet, si initialement la souche isolée de *Chitinophaga pinensis* est utilisée pour favoriser la croissance d'une culture agricole, elle peut lors d'un épisode de sécheresse soudain, en prévenir ces effets possiblement désastreux. L'inverse est d'ailleurs vrai. L'utilisation initiale d'une souche isolée de *Chitinophaga pinensis* pour prévenir et/ou traiter un épisode de sécheresse (stress hydrique) peut, après celui-ci, s'avérer promouvoir la reprise de la culture agricole et la croissance de celle-ci de manière à ce que le rendement final soit excellent. Autrement dit, un autre mode de réalisation concerne l'utilisation d'une souche isolée de *Chitinophaga pinensis* en tant qu'agent de lutte contre le stress hydrique et en tant qu'agent biofertilisant.

Par ailleurs et selon un autre mode de réalisation, l'invention a pour objet l'utilisation telle que décrite ci-dessus, ledit végétal appartenant à la famille des *Poaceae* tels que le blé, le riz, le maïs, l'orge, le millet, le seigle, l'épeautre, l'engrain ou petit épeautre, ou le sésame. En particulier, l'invention a pour objet l'utilisation telle que décrite ci-dessus, ledit végétal étant le blé, le riz, le maïs, l'orge, le millet, le seigle, l'épeautre, l'engrain ou petit épeautre, ou le sésame.

Selon un autre mode de réalisation, l'invention a pour objet l'utilisation telle que décrite ci-dessus, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Par « séquence d'acides nucléiques qui la code », on entend que ladite séquence d'acides nucléiques correspond à un cadre de lecture comprenant un codon initiateur et un codon terminateur, et dont la traduction par un ribosome permet d'aboutir à une protéine, *i.e.* un enchaînement d'acides aminés. A cet égard, il convient de noter que :

- la séquence d'acides nucléiques SEQ ID NO : 63 code pour l'enzyme de séquence SEQ ID NO: 71, laquelle correspond à la *Tryptophan 2-3 oxygenase* capable de catalyser la réaction enzymatique suivante :

$$L\text{-}tryptophan + O_2 = N\text{-}formyl\text{-}L\text{-}kynurenine$$

ladite enzyme étant impliquée dans la synthèse de l'indole-3-acétique (IAA) et dont l'activité peut être facilement testée (Yuzuru Ishimura, [52] L-tryptophan 2,3-dioxygenase (tryptophan pyrrolase) (pseudomonas fluorescens), Methods in Enzymology, Academic Press, Volume 17, Part A, 1970, Pages 429-434) ;

- la séquence d'acides nucléiques SEQ ID NO : 64 code pour l'enzyme de séquence SEQ ID NO : 72, laquelle correspond à la sous-unité bêta de la *Tryptophan synthase* capable de catalyser la réaction enzymatique suivante :

*(1S,2R)-1-C-(indol-3-yl)glycerol 3-phosphate + L-Serine = D-glyceraldehyde 3-phosphate + H2O + L-tryptophan*

ladite enzyme étant impliquée dans la synthèse du tryptophane et dont l'activité peut être facilement testée (Buller, Andrew R et al. "Directed evolution of the tryptophan synthase bêta-subunit for stand-alone function recapitulates allosteric activation." Proc Natl Acad Sci U S A. 2015 Nov 24; 112(47): 14599-14604.) ;

- la séquence d'acides nucléiques SEQ ID NO : 65 code pour l'enzyme de séquence SEQ ID NO : 73, laquelle correspond à sous-unité alpha de la *Tryptophan synthase* capable de catalyser la réaction enzymatique suivante :

*(1S,2R)-1-C-(indol-3-yl)glycerol 3-phosphate + L-Serine = D-glyceraldehyde 3-phosphate + H2O + L-tryptophan*

ladite enzyme étant impliquée dans la synthèse du tryptophane et dont l'activité peut être facilement testée (R.D. Mosteller, Improved method for measuring the catalytic activity of tryptophan synthase $\alpha$ subunit in cell extracts, Biochimie, Volume 72, Issue 12, 1990, Pages 881-884) ;

- la séquence d'acides nucléiques SEQ ID NO : 66 code pour l'enzyme de séquence SEQ ID NO : 74, laquelle correspond à l'*Indole-3-glycérol phosphate synthase TrpC* capable de catalyser la réaction enzymatique suivante :

*1-(2-carboxyphenylamino)-1-deoxy-D-ribulose 5-phosphate + H$^+$ = (1S,2R)-1-C-(indol-3-yl)glycerol 3-phosphate + CO$_2$ + H$_2$O*

ladite enzyme étant impliquée dans la synthèse du tryptophane et dont l'activité peut être facilement testée (Söderholm, Annika et al. "Structure and kinetics of indole-3-glycerol phosphate synthase from Pseudomonas aeruginosa: Decarboxylation is not essential for indole formation." The Journal of biological chemistry vol. 295,47 (2020): 15948-15956.) ;

- la séquence d'acides nucléiques SEQ ID NO : 67 code pour l'enzyme de séquence SEQ ID NO : 75, laquelle correspond à la *Phosphoglucomutase* capable de catalyser la réaction enzymatique suivante :

*bêta-D-glucose 1-phosphate = bêta-D-glucose 6-phosphate*

ladite enzyme étant impliqué dans la glycolyse et probablement la synthèse d' d'exopolysaccharides (EPS), et dont l'activité peut être facilement testée (H.U. Bergmeyer (Ed.), Methods of Enzymatic Analysis (Second edition), Academic Press (1974), pp. 798-801) ;

- la séquence d'acides nucléiques SEQ ID NO : 68 code pour l'enzyme de séquence SEQ ID NO : 76, laquelle correspond à la *Phosphomannomutase/Phosphoglucomutase* capable de catalyser la réaction enzymatique suivante :

*α-D-mannose 1-phosphate = D-mannose 6-phosphate*

ladite enzyme étant impliqué dans la synthèse d'exopolysaccharides (EPS) et dont l'activité peut être facilement testée (Körner C, Lehle L, von Figura K. Abnormal synthesis of mannose 1-phosphate derived carbohydrates in carbohydrate-deficient glycoprotein syndrome type I fibroblasts with phosphomannomutase deficiency. Glycobiology. 1998 Feb;8(2):165-71. doi: 10.1093/glycob/8.2.165. PMID: 9451026.) ;

- la séquence d'acides nucléiques SEQ ID NO : 69 code pour l'enzyme de séquence SEQ ID NO : 77, laquelle correspond à la *dTDP-glucose 4,6-dehydratase* capable de catalyser la réaction enzymatique suivante :

$$\textit{dTDP-\alpha-D-glucose = dTDP-4-dehydro-6-deoxy-\alpha-D-glucose + H}_2\textit{O}$$

ladite enzyme étant impliqué dans la synthèse d'exopolysaccharides (EPS) et dont l'activité peut être facilement testée (Okazaki R, Okazakit, Strominger JL, Michelson AM. Thymidine diphosphate 4-keto-6-deoxy-d-glucose, an intermediate in thymidine diphosphate L-rhamnose synthesis in Escherichia coli strains. J Biol Chem. 1962 Oct;237:3014-26. PMID: 13939805.) ; et

- la séquence d'acides nucléiques SEQ ID NO : 70 code pour la protéine de séquence SEQ ID NO : 78, laquelle correspond à l'*Alginate biosynthesis protein AlgA* capable de catalyser la réaction enzymatique suivante :

$$\textit{D-mannose 6-phosphate = D-fructose 6-phosphate}$$

ladite enzyme étant impliqué dans la synthèse d'exopolysaccharides (EPS) et dont l'activité peut être facilement testée (Shinabarger D, Berry A, May TB, Rothmel R, Fialho A, Chakrabarty AM. Purification and characterization of phosphomannose isomerase-guanosine diphospho-D-mannose pyrophosphorylase. A bifunctional enzyme in the alginate biosynthetic pathway of Pseudomonas aeruginosa. J Biol Chem. 1991 Feb 5;266(4):2080-8. PMID: 1846611.).

Par « pourcentage d'identité », on entend le pourcentage déterminé par comparaison directe de deux séquences de molécules d'acides nucléiques (ou d'acides aminés), en déterminant le nombre de résidus nucléiques identiques entre les deux séquences, puis en le divisant par le nombre de résidus nucléiques de la séquence la plus longue des deux, et en multipliant le résultat par 100. Des outils de comparaison de séquences tels que tels que les algorithmes de la plateforme BLAST ou en particulier le programme MatGat2.01 (Campanella, J. J., Bitincka, L., & Smalley, J. (2003). MatGAT: an application that generates similarity/identity matrices using protein or DNA sequences. BMC Bioinformatics, 4, 29.) peuvent aider à y parvenir. De plus et par « une identité d'au moins 84 % avec une séquence de référence », on entend que l'identité entre une séquence d'intérêt et une séquence de référence (choisie parmi les séquences SEQ ID NOs : 63 à 70) peut être d'au moins 84 %, au moins 85 %, au moins 86 %, au moins 87 %, au moins 88 %, au moins 89 %, au moins 90 %, au moins 91 %, au moins 92 %, au moins 93 %, au moins 94 %, au moins 95 %, au moins 96 %, au moins 97 %, au moins 98 %, ou d'au moins 99 %, sous réserve que ladite séquence d'intérêt conserve l'activité enzymatique de l'enzyme codée par la susdite séquence de référence. Cela signifie donc que la séquence d'intérêt (ou l'enzyme d'intérêt) est fonctionnelle et est capable de catalyser la même réaction enzymatique que le séquence de référence (ou enzyme de référence).

Selon un autre mode de réalisation, l'invention a pour objet l'utilisation telle que décrite ci-dessus, ladite souche isolée de *Chitinophaga pinensis* comprenant une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Selon un autre mode de réalisation, l'invention a pour objet l'utilisation telle que décrite ci-dessus, ladite souche isolée de *Chitinophaga pinensis* comprenant au moins une enzyme dont la séquence d'acides nucléiques qui la code est choisie parmi les séquences SEQ ID NOs : 63 à 70. En particulier, l'invention a pour objet l'utilisation telle que décrite ci-dessus, ladite souche isolée de *Chitinophaga pinensis* comprenant au moins une enzyme dont la séquence d'acides nucléiques correspond à la séquence SEQ ID NO : 69, laquelle code pour l'enzyme de séquence SEQ ID NO : 77.

Selon un autre mode de réalisation, l'invention a pour objet l'utilisation telle que décrite ci-dessus, ladite souche isolée de *Chitinophaga pinensis* comprenant une enzyme dont la séquence d'acides nucléiques qui la code est choisie parmi les séquences SEQ ID NOs : 63 à 70. En particulier, l'invention a pour objet l'utilisation telle que décrite ci-dessus, ladite souche isolée de *Chitinophaga pinensis* comprenant une enzyme dont la séquence d'acides nucléiques correspond à la séquence SEQ ID NO : 69, laquelle code pour l'enzyme de séquence SEQ ID NO : 77.

Selon un autre mode de réalisation, l'invention a pour objet l'utilisation telle que décrite ci-dessus, ladite souche isolée de *Chitinophaga pinensis* comprenant au moins deux enzymes dont les séquences d'acides nucléiques qui les codent ont une identité d'au moins 84 % avec une séquence de référence choisie parmi les séquences SEQ ID NOs : 63 à 70, lesdites au moins deux enzymes conservant l'activité enzymatique de l'enzyme codée par la susdite séquence de référence.

Selon un autre mode de réalisation, l'invention a pour objet l'utilisation telle que décrite ci-dessus, ladite souche isolée de *Chitinophaga pinensis* comprenant au moins deux enzymes dont les séquences d'acides nucléiques qui les codent sont choisies parmi les séquences SEQ ID NOs : 63 à 70.

Selon un autre mode de réalisation, l'invention a pour objet l'utilisation telle que décrite ci-dessus, ladite souche isolée de *Chitinophaga pinensis* comprenant au moins trois enzymes dont les séquences d'acides nucléiques qui les codent ont une identité d'au moins 84 % avec une séquence de référence choisie parmi les séquences SEQ ID NOs : 63 à 70, lesdites au moins trois enzymes conservant l'activité enzymatique de l'enzyme codée par la susdite séquence de référence.

Selon un autre mode de réalisation, l'invention a pour objet l'utilisation telle que décrite ci-dessus, ladite souche isolée de *Chitinophaga pinensis* comprenant au moins trois enzymes dont les séquences d'acides nucléiques qui les codent sont choisies parmi les séquences SEQ ID NOs : 63 à 70.

Selon un autre mode de réalisation, l'invention a pour objet l'utilisation telle que décrite ci-dessus, ladite souche isolée de *Chitinophaga pinensis* comprenant au moins quatre enzymes dont les séquences d'acides nucléiques qui les codent ont une identité d'au moins 84 % avec une séquence de référence choisie parmi les séquences SEQ ID NOs : 63 à 70, lesdites au moins quatre enzymes conservant l'activité enzymatique de l'enzyme codée par la susdite séquence de référence.

Selon un autre mode de réalisation, l'invention a pour objet l'utilisation telle que décrite ci-dessus, ladite souche isolée de *Chitinophaga pinensis* comprenant au moins quatre enzymes dont les séquences d'acides nucléiques qui les codent sont choisies parmi les séquences SEQ ID NOs : 63 à 70.

Selon un autre mode de réalisation, l'invention a pour objet l'utilisation telle que décrite ci-dessus, ladite souche isolée de *Chitinophaga pinensis* comprenant au moins cinq enzymes dont les séquences d'acides nucléiques qui les codent ont une identité d'au moins 84 % avec une séquence de référence choisie parmi les séquences SEQ ID NOs : 63 à 70, lesdites au moins cinq enzymes conservant l'activité enzymatique de l'enzyme codée par la susdite séquence de référence.

Selon un autre mode de réalisation, l'invention a pour objet l'utilisation telle que décrite ci-dessus, ladite souche isolée de *Chitinophaga pinensis* comprenant au moins cinq enzymes dont les séquences d'acides nucléiques qui les codent sont choisies parmi les séquences SEQ ID NOs : 63 à 70.

Selon un autre mode de réalisation, l'invention a pour objet l'utilisation telle que décrite ci-dessus, ladite souche isolée de *Chitinophaga pinensis* comprenant au moins six enzymes dont les séquences d'acides nucléiques qui les codent ont une identité d'au moins 84 % avec une séquence de référence choisie parmi les séquences SEQ ID NOs : 63 à 70, lesdites au moins six enzymes conservant l'activité enzymatique de l'enzyme codée par la susdite séquence de référence.

Selon un autre mode de réalisation, l'invention a pour objet l'utilisation telle que décrite ci-dessus, ladite souche isolée de *Chitinophaga pinensis* comprenant au moins six enzymes dont les séquences d'acides nucléiques qui les codent sont choisies parmi les séquences SEQ ID NOs : 63 à 70.

Selon un autre mode de réalisation, l'invention a pour objet l'utilisation telle que décrite ci-dessus, ladite souche isolée de *Chitinophaga pinensis* comprenant au moins sept enzymes dont les séquences d'acides nucléiques qui les codent ont une identité d'au moins 84 % avec une séquence de référence choisie parmi les séquences SEQ ID NOs : 63 à 70, lesdites au moins sept enzymes conservant l'activité enzymatique de l'enzyme codée par la susdite séquence de référence.

Selon un autre mode de réalisation, l'invention a pour objet l'utilisation telle que décrite ci-dessus, ladite souche isolée de *Chitinophaga pinensis* comprenant au moins sept enzymes dont les séquences d'acides nucléiques qui les codent sont choisies parmi les séquences SEQ ID NOs : 63 à 70.

Selon un autre mode de réalisation, l'invention a pour objet l'utilisation telle que décrite ci-dessus, ladite souche isolée de *Chitinophaga pinensis* comprenant au moins huit enzymes dont les séquences d'acides nucléiques qui les codent ont une identité d'au moins 84 % avec une séquence de référence choisie parmi les séquences SEQ ID NOs : 63 à 70, lesdites au moins huit enzymes conservant l'activité enzymatique de l'enzyme codée par la susdite séquence de référence.

Selon un autre mode de réalisation, l'invention a pour objet l'utilisation telle que décrite ci-dessus, ladite souche isolée de *Chitinophaga pinensis* comprenant au moins huit enzymes dont les séquences d'acides nucléiques qui les codent correspondent aux séquences SEQ ID NOs : 63 à 70.

Selon un autre mode de réalisation, l'invention a pour objet l'utilisation telle que décrite ci-dessus, ladite souche isolée de *Chitinophaga pinensis* comprenant au moins une séquence d'acides nucléiques génomique ayant une identité d'au moins 95 % avec une séquence choisie parmi les contigs de séquences SEQ ID NOs : 22 à 61.

Comme précédemment, par « pourcentage d'identité », on entend le pourcentage déterminé par comparaison directe de deux séquences de molécules d'acides nucléiques (ou d'acides aminés), en déterminant le nombre de résidus nucléiques identiques entre les deux séquences, puis en le divisant par le nombre de résidus nucléiques de la séquence la plus longue des deux, et en multipliant le résultat par 100. Des outils de comparaison de séquences tels que tels que les algorithmes de la plateforme BLAST ou en particulier le programme MatGat2.01 (Campanella, J. J., Bitincka, L., & Smalley, J. (2003). MatGAT: an application that generates similarity/identity matrices using protein or DNA sequences.

BMC Bioinformatics, 4, 29.) peuvent aider à y parvenir. De plus et par « une identité d'au moins 95 % avec une séquence de référence », on entend que l'identité entre une séquence d'intérêt et un contig de référence (choisie parmi les séquences SEQ ID NOs : 22 à 61) peut être d'au moins 95 %, au moins 96 %, au moins 97 %, au moins 98 %, ou d'au moins 99 %.

Selon un autre mode de réalisation, l'invention a pour objet l'utilisation telle que décrite ci-dessus, ladite souche isolée de *Chitinophaga pinensis* comprenant au moins une séquence d'acides nucléiques génomique choisie parmi les contigs de séquences SEQ ID NOs : 22 à 61.

Selon un autre mode de réalisation, l'invention a pour objet l'utilisation telle que décrite ci-dessus, ladite souche isolée de *Chitinophaga pinensis* comprenant au moins une séquence d'acides nucléiques génomique ayant une identité d'au moins 80 % avec une séquence choisie parmi les contigs de séquences SEQ ID NOs : 1 à 21.

Comme précédemment, par « pourcentage d'identité », on entend le pourcentage déterminé par comparaison directe de deux séquences de molécules d'acides nucléiques (ou d'acides aminés), en déterminant le nombre de résidus nucléiques identiques entre les deux séquences, puis en le divisant par le nombre de résidus nucléiques de la séquence la plus longue des deux, et en multipliant le résultat par 100. Des outils de comparaison de séquences tels que tels que les algorithmes de la plateforme BLAST ou en particulier le programme MatGat2.01 (Campanella, J. J., Bitincka, L., & Smalley, J. (2003). MatGAT: an application that generates similarity/identity matrices using protein or DNA sequences. BMC Bioinformatics, 4, 29.) peuvent aider à y parvenir. De plus et par « une identité d'au moins 80 % avec une séquence de référence », on entend que l'identité entre une séquence d'intérêt et un contig de référence (choisie parmi les séquences SEQ ID NOs : 1 à 21) peut être d'au moins 80 %, au moins 81 %, au moins 82 %, au moins 83 %, au moins 84 %, au moins 85 %, au moins 86 %, au moins 87 %, au moins 88 %, au moins 89 %, au moins 90 %, au moins 91 %, au moins 92 %, au moins 93 %, au moins 94 %, au moins 95 %, au moins 96 %, au moins 97 %, au moins 98 %, ou d'au moins 99 %.

Selon un autre mode de réalisation, l'invention a pour objet l'utilisation telle que décrite ci-dessus, ladite souche isolée de *Chitinophaga pinensis* comprenant au moins une séquence d'acides nucléiques génomique choisie parmi les contigs de séquences SEQ ID NOs : 1 à 21.

Selon un autre mode de réalisation, l'invention a pour objet l'utilisation telle que décrite ci-dessus, ladite souche isolée de *Chitinophaga pinensis* comprenant un ADN ribosomique (ADNr) 16S dont la séquence d'acides nucléiques a une identité d'au moins 99 % avec la séquence SEQ ID NO : 62.

Selon un autre mode de réalisation, l'invention a pour objet l'utilisation telle que décrite ci-dessus, ladite souche isolée de *Chitinophaga pinensis* comprenant un ADN ribosomique (ADNr) 16S dont la séquence d'acides nucléiques est la séquence SEQ ID NO : 62.

Selon un autre mode de réalisation, l'invention a pour objet l'utilisation telle que décrite ci-dessus, ladite souche isolée de *Chitinophaga pinensis* étant la souche ARDCPP14 déposée le 23 avril 2021 à la Collection Nationale de Cultures de Microorganismes (CNCM ; Institut Pasteur, 25-28 rue du Docteur Roux, F-75724 PARIS CEDEX 15) sous le numéro CNCM I-5676.

Selon un autre mode de réalisation, l'invention a pour objet l'utilisation telle que décrite ci-dessus, ladite souche isolée de *Chitinophaga pinensis* étant la souche ARDCPP14 déposée le 23 avril 2021 à la CNCM sous le numéro CNCM I-5676 et/ou un de ses mutants (fonctionnels).

Selon un autre mode de réalisation, l'invention a pour objet l'utilisation telle que décrite ci-dessus, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence, en particulier, ladite souche isolée de *Chitinophaga pinensis* étant la souche ARDCPP14 déposée le 23 avril 2021 à la CNCM sous le numéro CNCM I-5676.

Selon un deuxième aspect, l'invention a pour objet une composition phytostimulante comprenant :

- une souche isolée de *Chitinophaga pinensis* en tant que principe actif ; ou
- une souche isolée de *Chitinophaga pinensis* en tant que premier principe actif en association avec au moins un autre principe actif choisi parmi :

  ◦ un autre agent de biostimulation ;
  ◦ un produit phytopharmaceutique de biocontrôle ;
  ◦ un engrais ; et
  ◦ leurs mélanges,

en particulier, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Par « principe actif », on entend le (co-)formulant ou les (co-)formulants qui dans une composition possède l'effet souhaité (amélioration de l'assimilation des minéraux et/ou tolérance au stress hydrique).

Par « un autre agent de biostimulation », on entend que cet autre agent de biostimulation n'est pas la souche isolée *Chitinophaga pinensis* selon l'invention. Par exemple, il peut être une souche de *Bacillus spp.* ou une souche d'*Ensifer spp* ou une souche de *Chitinophaga spp.* (différente de celle de l'invention).

Par « produit phytopharmaceutique de biocontrôle », on entend un (co-)formulant chimique et/ou biologique (e.g. bactérie, champignon) qui peut interférer avec la croissance et/ou la survie d'agents pathogènes de plantes.

Par « engrais », on entend les engrais chimiques, ou engrais minéraux, lesquels sont des fertilisants déversés sur les cultures (agricoles) le plus souvent par épandage et qui sont destinés à améliorer la quantité et la qualité des rendements agricoles, horticoles et sylvicoles. Par « engrais », on entend également les engrais organiques issus de matière vivante (animale, végétale, fongique, bactérienne...), comme le fumier.

Par « leurs mélanges », on entend que ledit au moins un autre principe actif peut être une combinaison entre :

- un autre agent de biostimulation et un produit phytopharmaceutique de biocontrôle ;
- un autre agent de biostimulation et un engrais ;
- un produit phytopharmaceutique de biocontrôle et un engrais ; ou
- un autre agent de biostimulation, un produit phytopharmaceutique de biocontrôle et un engrais.

Autrement dit, un autre mode de réalisation concerne la composition phytostimulante telle que décrite ci-dessus comprenant :

- une souche isolée de *Chitinophaga pinensis* en tant que principe actif ; ou
- une souche isolée de *Chitinophaga pinensis* en association avec au moins un autre agent de biostimulation en tant que principe actif ; ou
- une souche isolée de *Chitinophaga pinensis* en association avec au moins un produit phytopharmaceutique de biocontrôle en tant que principe actif ; ou
- une souche isolée de *Chitinophaga pinensis* en association avec au moins un engrais en tant que principe actif ; ou
- une souche isolée de *Chitinophaga pinensis* en association avec au moins un autre agent de biostimulation et avec au moins un produit phytopharmaceutique de biocontrôle en tant que principe actif ; ou
- une souche isolée de *Chitinophaga pinensis* en association avec au moins un autre agent de biostimulation et avec au moins un engrais en tant que principe actif ; ou
- une souche isolée de *Chitinophaga pinensis* en association avec au moins un un produit phytopharmaceutique de biocontrôle et avec au moins un engrais en tant que principe actif ; ou
- une souche isolée de *Chitinophaga pinensis* en association avec au moins un autre agent de biostimulation, avec au moins un produit phytopharmaceutique de biocontrôle et avec au moins un engrais en tant que principe actif.

Par ailleurs et dans la mesure où il est fait référence à « au moins un autre agent de biostimulation », « au moins un produit phytopharmaceutique de biocontrôle » et « au moins un engrais », il est entendu que la composition la composition phytostimulante telle que décrite ci-dessus peut comprendre au moins deux, au moins trois voire au moins quatre autres agents de biostimulation et/ou produits phytopharmaceutiques de biocontrôle et/ou engrais.

Selon un autre mode de réalisation, l'invention concerne également la composition phytostimulante telle que décrite ci-dessus, comprenant une quantité efficace de la souche isolée de *Chitinophaga pinensis* comprise de $10^6$ à $10^{12}$ UFC/g, plus particulièrement comprise de $10^8$ à $10^{10}$ UFC/g, ladite composition phytostimulante étant éventuellement obtenue après une étape de préparation (e.g. dilution par mise en suspension ou mélange) la rendant prête à l'épandage.

Par « quantité efficace [...] comprise de $10^6$ à $10^{12}$ UFC/g », on entend le nombre de bactéries capables de former une colonie sur milieu de culture (UFC= unité formant colonie) par g (gramme) de produit. De plus, l'expression « de $10^6$ à $10^{12}$ UFC/g » peut également signifier de $10^6$ à $10^{11}$ UFC/g, de $10^6$ à $10^{10}$ UFC/g, de $10^6$ à $10^9$ UFC/g, de $10^6$ à $10^8$ UFC/g, de $10^6$ à $10^7$ UFC/g, de $10^7$ à $10^{12}$ UFC/g, de $10^8$ à $10^{12}$ UFC/g, de $10^9$ à $10^{12}$ UFC/g, de $10^{10}$ à $10^{12}$ UFC/g, de $10^{11}$ à $10^{12}$ UFC/g, de $10^7$ à $10^{11}$ UFC/g, ou de $10^8$ à $10^{10}$ UFC/g.

Par ailleurs et dans la mesure où la composition phytostimulante telle que décrite ci-dessus peut être obtenue après une étape de préparation, on comprend qu'un objet de l'invention est une composition phytostimulante concentrée. En effet, la composition phytostimulante telle que décrite ci-dessus peut se présenter à la base (à l'achat) sous la forme d'une composition solide concentrée ou liquide concentrée, laquelle peut, par exemple, être obtenue grâce à une formulation particulière d'un des principes actifs (*cf. supra*). Aussi et par « composition [...] concentrée », on entend une composition phytostimulante dans laquelle au moins la concentration de la souche isolée de *Chitinophaga pinensis* est supérieure à une quantité efficace de $10^{12}$ UFC/g. Autrement dit, un aspect particulier de l'invention concerne la composition phytostimulante telle que décrite ci-dessus comprenant au moins la souche isolée de *Chitinophaga pinensis* en tant que principe actif, ladite composition phytostimulante se présentant sous la forme d'une composition solide concentrée ou liquide concentrée dans laquelle la concentration de la souche isolée de *Chitinophaga pinensis* est supérieure à une quantité efficace de $10^{12}$ UFC/g. Par « concentration [...] supérieure à une quantité efficace de $10^{12}$ UFC/g », on entend une concentration strictement supérieure à $10^{12}$ UFC/g, laquelle également être (strictement) supérieure à $10^{13}$ UFC/g, $10^{14}$ UFC/g, $10^{15}$ UFC/g voire $10^{16}$ UFC/g.

De plus, comme la composition phytostimulante de l'invention comprend au moins la souche isolée de *Chitinophaga pinensis* de l'invention comme principe actif, les caractéristiques particulières de celle-ci peuvent s'y appliquer. Autrement dit et selon un autre mode de réalisation, l'invention a pour objet la composition phytostimulante telle que décrite ci-dessus, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant au moins une enzyme dont la séquence d'acides nucléiques qui la code est choisie parmi les séquences SEQ ID NOs : 63 à 70. En particulier, l'invention a pour objet la composition phytostimulante telle que décrite ci-dessus, ladite souche isolée de *Chitinophaga pinensis* comprenant au moins une enzyme dont la séquence d'acides nucléiques correspond à la séquence SEQ ID NO : 69, laquelle code pour l'enzyme de séquence SEQ ID NO : 77.

Selon un autre mode de réalisation, l'invention a pour objet la composition phytostimulante telle que décrite ci-dessus, ladite souche isolée de *Chitinophaga pinensis* comprenant une enzyme dont la séquence d'acides nucléiques qui la code est choisie parmi les séquences SEQ ID NOs : 63 à 70. En particulier, l'invention a pour objet la composition phytostimulante telle que décrite ci-dessus, ladite souche isolée de *Chitinophaga pinensis* comprenant une enzyme dont la séquence d'acides nucléiques correspond à la séquence SEQ ID NO : 69, laquelle code pour l'enzyme de séquence SEQ ID NO : 77.

Selon un autre mode de réalisation, l'invention a pour objet la composition phytostimulante telle que décrite ci-dessus, ladite souche isolée de *Chitinophaga pinensis* comprenant au moins deux enzymes dont les séquences d'acides nucléiques qui les codent ont une identité d'au moins 84 % avec une séquence de référence choisie parmi les séquences SEQ ID NOs : 63 à 70, lesdites au moins deux enzymes conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant au moins deux enzymes dont les séquences d'acides nucléiques qui les codent sont choisies parmi les séquences SEQ ID NOs : 63 à 70.

Selon un autre mode de réalisation, l'invention a pour objet la composition phytostimulante telle que décrite ci-dessus, ladite souche isolée de *Chitinophaga pinensis* comprenant au moins trois enzymes dont les séquences d'acides nucléiques qui les codent ont une identité d'au moins 84 % avec une séquence de référence choisie parmi les séquences SEQ ID NOs : 63 à 70, lesdites au moins trois enzymes conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant au moins trois enzymes dont les séquences d'acides nucléiques qui les codent sont choisies parmi les séquences SEQ ID NOs : 63 à 70.

Selon un autre mode de réalisation, l'invention a pour objet la composition phytostimulante telle que décrite ci-dessus, ladite souche isolée de *Chitinophaga pinensis* comprenant au moins quatre enzymes dont les séquences d'acides nucléiques qui les codent ont une identité d'au moins 84 % avec une séquence de référence choisie parmi les séquences SEQ ID NOs : 63 à 70, lesdites au moins quatre enzymes conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant au moins quatre enzymes dont les séquences d'acides nucléiques qui les codent sont choisies parmi les séquences SEQ ID NOs : 63 à 70.

Selon un autre mode de réalisation, l'invention a pour objet la composition phytostimulante telle que décrite ci-dessus, ladite souche isolée de *Chitinophaga pinensis* comprenant au moins cinq enzymes dont les séquences d'acides nucléiques qui les codent ont une identité d'au moins 84 % avec une séquence de référence choisie parmi les séquences SEQ ID NOs : 63 à 70, lesdites au moins cinq enzymes conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant au moins cinq enzymes dont les séquences d'acides nucléiques qui les codent sont choisies parmi les séquences SEQ ID NOs : 63 à 70.

Selon un autre mode de réalisation, l'invention a pour objet la composition phytostimulante telle que décrite ci-dessus, ladite souche isolée de *Chitinophaga pinensis* comprenant au moins six enzymes dont les séquences d'acides nucléiques qui les codent ont une identité d'au moins 84 % avec une séquence de référence choisie parmi les séquences SEQ ID NOs : 63 à 70, lesdites au moins six enzymes conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant au moins six enzymes dont les séquences d'acides nucléiques qui les codent sont choisies parmi les séquences SEQ ID NOs : 63 à 70.

Selon un autre mode de réalisation, l'invention a pour objet la composition phytostimulante telle que décrite ci-dessus, ladite souche isolée de *Chitinophaga pinensis* comprenant au moins sept enzymes dont les séquences d'acides nucléiques qui les codent ont une identité d'au moins 84 % avec une séquence de référence choisie parmi les séquences SEQ ID NOs : 63 à 70, lesdites au moins sept enzymes conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant au moins sept enzymes dont les séquences d'acides nucléiques qui les codent sont choisies parmi les séquences SEQ ID NOs : 63 à 70.

Selon un autre mode de réalisation, l'invention a pour objet la composition phytostimulante telle que décrite ci-dessus, ladite souche isolée de *Chitinophaga pinensis* comprenant au moins huit enzymes dont les séquences d'acides nucléiques qui les codent ont une identité d'au moins 84 % avec une séquence de référence choisie parmi les séquences SEQ ID NOs : 63 à 70, lesdites au moins huit enzymes conservant l'activité enzymatique de l'enzyme codée par la susdite séquence de référence.

Selon un autre mode de réalisation, l'invention a pour objet la composition phytostimulante telle que décrite ci-dessus, ladite souche isolée de *Chitinophaga pinensis* comprenant au moins huit enzymes dont les séquences d'acides nucléiques qui les codent correspondent aux séquences SEQ ID NOs : 63 à 70.

Selon un autre mode de réalisation, l'invention a pour objet la composition phytostimulante telle que décrite ci-dessus, ladite souche isolée de *Chitinophaga pinensis* comprenant au moins une séquence d'acides nucléiques génomique ayant une identité d'au moins 95 % avec une séquence choisie parmi les contigs de séquences SEQ ID NOs : 22 à 61.

Selon un autre mode de réalisation, l'invention a pour objet la composition phytostimulante telle que décrite ci-dessus, ladite souche isolée de *Chitinophaga pinensis* comprenant au moins une séquence d'acides nucléiques génomique choisie parmi les contigs de séquences SEQ ID NOs : 22 à 61.

Selon un autre mode de réalisation, l'invention a pour objet la composition phytostimulante telle que décrite ci-dessus, ladite souche isolée de *Chitinophaga pinensis* comprenant au moins une séquence d'acides nucléiques génomique ayant une identité d'au moins 80 % avec une séquence choisie parmi les contigs de séquences SEQ ID NOs : 1 à 21.

Selon un autre mode de réalisation, l'invention a pour objet la composition phytostimulante telle que décrite ci-dessus, ladite souche isolée de *Chitinophaga pinensis* comprenant au moins une séquence d'acides nucléiques génomique choisie parmi les contigs de séquences SEQ ID NOs : 1 à 21.

Selon un autre mode de réalisation, l'invention a pour objet la composition phytostimulante telle que décrite ci-dessus, ladite souche isolée de *Chitinophaga pinensis* comprenant un ADN ribosomique (ADNr) 16S dont la séquence d'acides nucléiques a une identité d'au moins 99 % avec la séquence SEQ ID NO : 62.

Selon un autre mode de réalisation, l'invention a pour objet la composition phytostimulante telle que décrite ci-dessus, ladite souche isolée de *Chitinophaga pinensis* comprenant un ADN ribosomique (ADNr) 16S dont la séquence d'acides nucléiques est la séquence SEQ ID NO : 62.

Selon un autre mode de réalisation, l'invention a pour objet la composition phytostimulante telle que décrite ci-dessus, ladite souche isolée de *Chitinophaga pinensis* étant la souche ARDCPP14 déposée le 23 avril 2021 à la Collection Nationale de Cultures de Microorganismes (CNCM ; Institut Pasteur, 25-28 rue du Docteur Roux, F-75724 PARIS CEDEX 15) sous le numéro CNCM I-5676.

Selon un autre mode de réalisation, l'invention a pour objet la composition phytostimulante telle que décrite ci-dessus, ladite souche isolée de *Chitinophaga pinensis* étant la souche ARDCPP14 déposée le 23 avril 2021 à la CNCM sous le numéro CNCM I-5676 et/ou un de ses mutants (fonctionnels).

Par ailleurs et au vu de ce qui précède, on comprend qu'un objet de l'invention concerne la composition phytostimulante telle que décrite ci-dessus comprenant :

- une souche isolée de *Chitinophaga pinensis* en tant que principe actif ; ou
- une souche isolée de *Chitinophaga pinensis* en tant que premier principe actif en association avec au moins un autre principe actif choisi parmi :

- ◦ un autre agent de biostimulation ;
- ◦ un produit phytopharmaceutique de biocontrôle ;
- ◦ un engrais ; et
- ◦ leurs mélanges,

en particulier, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence,

en particulier, ladite souche isolée de *Chitinophaga pinensis* étant la souche ARDCPP14 déposée le 23 avril 2021 à la CNCM sous le numéro CNCM I-5676.

Selon un autre aspect, l'invention concerne une composition phytostimulante d'enrobage comprenant une souche isolée de *Chitinophaga pinensis* et éventuellement au moins un agent de fixation.

Par « agent de fixation », on entend un agent chimique ou naturel lequel permet de coller la composition phytostimulante à une graine de végétal de manière à enrober ladite graine de végétal. Parmi les agents de fixation disponibles, on retrouve notamment la carboxymethyl cellulose (CMC) et la gomme arabique.

Par « une souche isolée de *Chitinophaga pinensis* », on entend en particulier la souche telle que décrite précédemment. De fait, on comprend qu'un autre objet de l'invention concerne la composition phytostimulante d'enrobage telle que décrite ci-dessus, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant au moins une enzyme dont la séquence d'acides nucléiques qui la code est choisie parmi les séquences SEQ ID NOs : 63 à 70. En particulier, l'invention a pour objet la composition phytostimulante telle que décrite ci-dessus, ladite souche isolée de *Chitinophaga pinensis* comprenant au moins une enzyme dont la séquence d'acides nucléiques correspond à la séquence SEQ ID NO : 69, laquelle code pour l'enzyme de séquence SEQ ID NO : 77.

Selon un autre mode de réalisation, l'invention a pour objet la composition phytostimulante d'enrobage telle que décrite ci-dessus, ladite souche isolée de *Chitinophaga pinensis* comprenant une enzyme dont la séquence d'acides nucléiques qui la code est choisie parmi les séquences SEQ ID NOs : 63 à 70. En particulier, l'invention a pour objet la composition phytostimulante telle que décrite ci-dessus, ladite souche isolée de *Chitinophaga pinensis* comprenant une enzyme dont la séquence d'acides nucléiques correspond à la séquence SEQ ID NO : 69, laquelle code pour l'enzyme de séquence SEQ ID NO : 77.

Selon un autre mode de réalisation, l'invention a pour objet la composition phytostimulante d'enrobage telle que décrite ci-dessus, ladite souche isolée de *Chitinophaga pinensis* comprenant au moins deux enzymes dont les séquences d'acides nucléiques qui les codent ont une identité d'au moins 84 % avec une séquence de référence choisie parmi les séquences SEQ ID NOs : 63 à 70, lesdites au moins deux enzymes conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant au moins deux enzymes dont les séquences d'acides nucléiques qui les codent sont choisies parmi les séquences SEQ ID NOs : 63 à 70.

Selon un autre mode de réalisation, l'invention a pour objet la composition phytostimulante d'enrobage telle que décrite ci-dessus, ladite souche isolée de *Chitinophaga pinensis* comprenant au moins trois enzymes dont les séquences d'acides nucléiques qui les codent ont une identité d'au moins 84 % avec une séquence de référence choisie parmi les séquences SEQ ID NOs : 63 à 70, lesdites au moins trois enzymes conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant au moins trois enzymes dont les séquences d'acides nucléiques qui les codent sont choisies parmi les séquences SEQ ID NOs : 63 à 70.

Selon un autre mode de réalisation, l'invention a pour objet la composition phytostimulante d'enrobage telle que décrite ci-dessus, ladite souche isolée de *Chitinophaga pinensis* comprenant au moins quatre enzymes dont les séquences d'acides nucléiques qui les codent ont une identité d'au moins 84 % avec une séquence de référence choisie parmi les séquences SEQ ID NOs : 63 à 70, lesdites au moins quatre enzymes conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant au moins quatre enzymes dont les séquences d'acides nucléiques qui les codent sont choisies parmi les séquences SEQ ID NOs : 63 à 70.

Selon un autre mode de réalisation, l'invention a pour objet la composition phytostimulante d'enrobage telle que décrite ci-dessus, ladite souche isolée de *Chitinophaga pinensis* comprenant au moins cinq enzymes dont les séquences d'acides nucléiques qui les codent ont une identité d'au moins 84 % avec une séquence de référence choisie parmi les séquences SEQ ID NOs : 63 à 70, lesdites au moins cinq enzymes conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant au moins cinq enzymes dont les séquences d'acides nucléiques qui les codent sont choisies parmi les séquences SEQ ID NOs : 63 à 70.

Selon un autre mode de réalisation, l'invention a pour objet la composition phytostimulante d'enrobage telle que décrite ci-dessus, ladite souche isolée de *Chitinophaga pinensis* comprenant au moins six enzymes dont les séquences d'acides nucléiques qui les codent ont une identité d'au moins 84 % avec une séquence de référence choisie parmi les séquences SEQ ID NOs : 63 à 70, lesdites au moins six enzymes conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant au moins six enzymes dont les séquences d'acides nucléiques qui les codent sont choisies parmi les séquences SEQ ID NOs : 63 à 70.

Selon un autre mode de réalisation, l'invention a pour objet la composition phytostimulante d'enrobage telle que décrite ci-dessus, ladite souche isolée de *Chitinophaga pinensis* comprenant au moins sept enzymes dont les séquences d'acides nucléiques qui les codent ont une identité d'au moins 84 % avec une séquence de référence choisie parmi les séquences SEQ ID NOs : 63 à 70, lesdites au moins sept enzymes conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant au moins sept enzymes dont les séquences d'acides nucléiques qui les codent sont choisies parmi les séquences SEQ ID NOs : 63 à 70.

Selon un autre mode de réalisation, l'invention a pour objet la composition phytostimulante d'enrobage telle que décrite ci-dessus, ladite souche isolée de *Chitinophaga pinensis* comprenant au moins huit enzymes dont les séquences d'acides nucléiques qui les codent ont une identité d'au moins 84 % avec une séquence de référence choisie parmi les séquences SEQ ID NOs : 63 à 70, lesdites au moins huit enzymes conservant l'activité enzymatique de l'enzyme codée par la susdite séquence de référence.

Selon un autre mode de réalisation, l'invention a pour objet la composition phytostimulante d'enrobage telle que décrite ci-dessus, ladite souche isolée de *Chitinophaga pinensis* comprenant au moins huit enzymes dont les séquences d'acides nucléiques qui les codent correspondent aux séquences SEQ ID NOs : 63 à 70.

Selon un autre mode de réalisation, l'invention a pour objet la composition phytostimulante d'enrobage telle que décrite ci-dessus, ladite souche isolée de *Chitinophaga pinensis* comprenant au moins une séquence d'acides nucléiques génomique ayant une identité d'au moins 95 % avec une séquence choisie parmi les contigs de séquences SEQ ID NOs : 22 à 61.

Selon un autre mode de réalisation, l'invention a pour objet la composition phytostimulante d'enrobage telle que décrite ci-dessus, ladite souche isolée de *Chitinophaga pinensis* comprenant au moins une séquence d'acides nucléiques génomique choisie parmi les contigs de séquences SEQ ID NOs : 22 à 61.

Selon un autre mode de réalisation, l'invention a pour objet la composition phytostimulante d'enrobage telle que décrite ci-dessus, ladite souche isolée de *Chitinophaga pinensis* comprenant au moins une séquence d'acides nucléiques génomique ayant une identité d'au moins 80 % avec une séquence choisie parmi les contigs de séquences SEQ ID NOs : 1 à 21.

Selon un autre mode de réalisation, l'invention a pour objet la composition phytostimulante d'enrobage telle que décrite ci-dessus, ladite souche isolée de *Chitinophaga pinensis* comprenant au moins une séquence d'acides nucléiques génomique choisie parmi les contigs de séquences SEQ ID NOs : 1 à 21.

Selon un autre mode de réalisation, l'invention a pour objet la composition phytostimulante d'enrobage telle que décrite ci-dessus, ladite souche isolée de *Chitinophaga pinensis* comprenant un ADN ribosomique (ADNr) 16S dont la séquence d'acides nucléiques a une identité d'au moins 99 % avec la séquence SEQ ID NO : 62.

Selon un autre mode de réalisation, l'invention a pour objet la composition phytostimulante d'enrobage telle que décrite ci-dessus, ladite souche isolée de *Chitinophaga pinensis* comprenant un ADN ribosomique (ADNr) 16S dont la séquence d'acides nucléiques est la séquence SEQ ID NO : 62.

Selon un autre mode de réalisation, l'invention a pour objet la composition phytostimulante d'enrobage telle que décrite ci-dessus, ladite souche isolée de *Chitinophaga pinensis* étant la souche ARDCPP14 déposée le 23 avril 2021 à la Collection Nationale de Cultures de Microorganismes (CNCM ; Institut Pasteur, 25-28 rue du Docteur Roux, F-75724 PARIS CEDEX 15) sous le numéro CNCM I-5676.

Selon un autre mode de réalisation, l'invention a pour objet la composition phytostimulante d'enrobage telle que décrite ci-dessus, ladite souche isolée de *Chitinophaga pinensis* étant la souche ARDCPP14 déposée le 23 avril 2021 à la CNCM sous le numéro CNCM I-5676 et/ou un de ses mutants (fonctionnels).

Par ailleurs et au vu de ce qui précède, on comprend qu'un objet de l'invention concerne la composition phytostimulante d'enrobage telle que décrite ci-dessus comprenant une souche isolée de *Chitinophaga pinensis* et éventuellement au moins un agent de fixation, en particulier, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence,

en particulier, ladite souche isolée de *Chitinophaga pinensis* étant la souche ARDCPP14 déposée le 23 avril 2021 à la CNCM sous le numéro CNCM I-5676.

Selon un autre mode de réalisation, l'invention a également pour objet la composition phytostimulante d'enrobage telle que décrite ci-dessus, comprenant en outre au moins un co-formulant choisi parmi :

- le glycérol ;
- la gomme arabique ;
- le polyvinylpyrrolidone (PVP) ;
- l'acide ascorbique ;
- lait écrémé ; et
- leurs mélanges.

Par « leurs mélanges », on entend on entend que ledit au moins un co-formulant peut être, par exemple, une combinaison entre :

- le glycérol et la gomme arabique et/ou le polyvinylpyrrolidone (PVP) ;
- la gomme arabique et le glycérol et/ou le polyvinylpyrrolidone (PVP) ;
- le polyvinylpyrrolidone (PVP) et la gomme arabique et/ou le glycérol ;
- l'acide ascorbique et le lait écrémé ;
- le glycérol et la gomme arabique et/ou le polyvinylpyrrolidone (PVP), et l'acide ascorbique et/ou le lait écrémé ;
- la gomme arabique et le glycérol et/ou le polyvinylpyrrolidone (PVP), et l'acide ascorbique et/ou le lait écrémé ;
- le polyvinylpyrrolidone (PVP) et la gomme arabique et/ou le glycérol ; , et l'acide ascorbique et/ou le lait écrémé ; ou
- le glycérol, la gomme arabique, le polyvinylpyrrolidone (PVP), l'acide ascorbique et le lait écrémé.

Autrement dit, un autre mode de réalisation concerne la composition phytostimulante d'enrobage telle que décrite ci-dessus comprenant en outre un co-formulant choisi parmi :

- le mélange comprenant du glycérol, de la gomme arabique et/ou du polyvinylpyrrolidone (PVP) ;
- le mélange comprenant de la gomme arabique, du glycérol et/ou du polyvinylpyrrolidone (PVP) ;
- le mélange comprenant le mélange comprenant du polyvinylpyrrolidone (PVP), de la gomme arabique et/ou du glycérol ;
- le mélange comprenant de l'acide ascorbique et du lait écrémé ;
- le mélange comprenant du glycérol, de la gomme arabique et/ou du polyvinylpyrrolidone (PVP), et de l'acide ascorbique et/ou du lait écrémé ;
- le mélange comprenant de la gomme arabique, du glycérol et/ou du polyvinylpyrrolidone (PVP), et de l'acide ascorbique et/ou du lait écrémé ;
- le mélange comprenant du polyvinylpyrrolidone (PVP), de la gomme arabique et/ou du glycérol, et de l'acide ascorbique et/ou du lait écrémé ; et
- le mélange comprenant du glycérol, de la gomme arabique, du polyvinylpyrrolidone (PVP), de l'acide ascorbique et du lait écrémé.

En particulier, un autre mode de réalisation concerne la composition phytostimulante d'enrobage telle que décrite ci-dessus, dans laquelle :

- la concentration de glycérol est comprise de 1 % m/m à 4 % m/m ; et/ou
- la concentration de gomme arabique est comprise de 2,5 % m/m à 10 % m/m ; et/ou
- la concentration de polyvinylpyrrolidone (PVP) est comprise de 1 % m/m à 4 % m/m ; et/ou
- la concentration d'acide ascorbique est comprise de 0,01 % m/m à 0,04 % m/m ; et/ou
- la concentration de lait écrémé est comprise de 2 % m/m à 5 % m/m.

En particulier, un autre mode de réalisation concerne la composition phytostimulante d'enrobage telle que décrite ci-dessus, dans laquelle :

- la concentration de glycérol est de 2 % m/m ; et/ou
- la concentration de gomme arabique est de 5 % m/m ; et/ou
- la concentration de polyvinylpyrrolidone (PVP) est de 2 % m/m ; et/ou
- la concentration d'acide ascorbique est de 0,02 % m/m ; et/ou
- la concentration de lait écrémé est de 3 % m/m.

Sur ce point, il convient de noter que parmi ces co-formulants se trouvent : des agents protecteurs (l'acide ascorbique est un antioxydant et le polyvinylpyrrolidone (PVP) protège notamment de la chaleur), des supports (ou « *carrier* » en anlglais) et des éléments nutritifs (*e.g.* talc, vermiculite, tourbe, lait écrémé, glycérol, saccharose). Toutefois certains éléments peuvent avoir un double rôle. Par exemple, le glycérol est un élément nutritif et un agent protecteur contre la dessication, et la gomme arabique est souvent considérée comme un agent de fixation mais aussi parfois comme un support (« *carrier* »)*.*

Au vu de ce qui précède, on comprend qu'un autre aspect de l'invention a pour objet une semence enrobée comprenant une graine de végétal, ladite graine de végétal étant enrobée par la composition phytostimulante d'enrobage telle que décrite ci-dessus,

en particulier ladite graine de végétal appartenant à un végétal de la famille des *Poaceae* tels que le blé, le riz, le maïs, l'orge, le millet, le seigle, l'épeautre, l'engrain ou petit épeautre, ou le sésame.

Selon un autre mode de réalisation, l'invention a pour objet pour objet la semence enrobée telle que décrite ci-dessus, ladite graine de végétal appartenant à la famille des *Poaceae* tels que le blé, le riz, le maïs, l'orge, le millet, le seigle, l'épeautre, l'engrain ou petit épeautre, ou le sésame. En particulier, l'invention a pour objet pour objet la semence enrobée telle que décrite ci-dessus, ladite graine de végétal appartenant à un végétal choisi parmi le blé, le riz, le maïs, l'orge, le millet, le seigle, l'épeautre, l'engrain ou petit épeautre, et le sésame.

Selon un autre mode de réalisation, l'invention a également pour objet la semence enrobée telle que décrite ci-dessus, dans laquelle la concentration de la souche isolée de *Chitinophaga pinensis* est comprise de $10^6$ à $10^8$ UFC/graine.

De plus, comme la semence enrobée de l'invention comprend la souche isolée de *Chitinophaga pinensis* de l'invention *via* l'enrobage lequel est réalisé à partir/grâce à la composition phytostimulante d'enrobage telle que décrite ci-dessus, les caractéristiques particulières de celle-ci peuvent s'y appliquer (*cf. supra*). Autrement dit et selon un autre mode de réalisation, l'invention a pour objet la semence enrobée telle que décrite ci-dessus, dans laquelle ladite souche isolée de *Chitinophaga pinensis* comprend au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence. *Etc.*

Selon un autre aspect de l'invention, celle-ci a pour objet un procédé de protection et/ou de traitement du stress hydrique subi par un végétal comprenant l'application de la composition phytostimulante telle que décrite ci-dessus sur au moins une partie dudit végétal ou du sol à proximité dudit végétal, en particulier ladite partie dudit végétal étant une feuille, un fruit ou une graine, et en particulier ledit végétal appartenant à la famille des *Poaceae* tels que le blé, le riz, le maïs, l'orge, le millet, le seigle, l'épeautre, l'engrain ou petit épeautre, ou le sésame.

Selon un autre mode de réalisation, l'invention a pour objet un procédé de protection du stress hydrique subi par un végétal comprenant l'application de la composition phytostimulante telle que décrite ci-dessus sur au moins une partie dudit végétal ou du sol à proximité dudit végétal, en particulier ladite partie dudit végétal étant une feuille, un fruit ou une graine, et en particulier ledit végétal appartenant à la famille des *Poaceae* tels que le blé, le riz, le maïs, l'orge, le millet, le seigle, l'épeautre, l'engrain ou petit épeautre, ou le sésame.

Selon un autre mode de réalisation, l'invention a pour objet un procédé de traitement du stress hydrique subi par un végétal comprenant l'application de la composition phytostimulante telle que décrite ci-dessus sur au moins une partie dudit végétal ou du sol à proximité dudit végétal, en particulier ladite partie dudit végétal étant une feuille, un fruit ou une graine, et en particulier ledit végétal appartenant à la famille des *Poaceae* tels que le blé, le riz, le maïs, l'orge, le millet, le seigle, l'épeautre, l'engrain ou petit épeautre, ou le sésame.

Selon un autre mode de réalisation, l'invention a pour objet un procédé de protection et de traitement du stress hydrique subi par un végétal comprenant l'application de la composition phytostimulante telle que décrite ci-dessus sur au moins une partie dudit végétal ou du sol à proximité dudit végétal,

en particulier ladite partie dudit végétal étant une feuille, un fruit ou une graine, et
en particulier ledit végétal appartenant à la famille des *Poaceae* tels que le blé, le riz, le maïs, l'orge, le millet, le seigle, l'épeautre, l'engrain ou petit épeautre, ou le sésame.

Par « procédé de protection du stress hydrique subi par un végétal », on entend un procédé qui une fois mis en œuvre réduit l'impact négatif du manque d'eau sur la croissance et le développement du végétal.

Par « procédé de traitement du stress hydrique subi par un végétal », on entend un procédé qui une fois mis en œuvre permet au végétal de mieux surmonter les conséquences d'un manque d'eau.

Par ailleurs et selon un autre mode de réalisation, l'invention a pour objet le procédé tel que décrit ci-dessus, ladite partie dudit végétal étant une feuille, un fruit ou une graine.

Selon un autre mode de réalisation, l'invention a également pour objet le procédé tel que décrit ci-dessus, ledit végétal appartenant à la famille des *Poaceae* tels que le blé, le riz, le maïs, l'orge, le millet, le seigle, l'épeautre, l'engrain ou petit épeautre, ou le sésame. En particulier, l'invention a pour objet le procédé tel que décrit ci-dessus, ledit végétal étant choisi parmi le blé, le riz, le maïs, l'orge, le millet, le seigle, l'épeautre, l'engrain ou petit épeautre, et le sésame.

Selon un autre aspect de l'invention, celle-ci a pour objet un procédé pour stimuler l'assimilation des minéraux par un végétal comprenant l'application de la composition phytostimulante telle que décrite ci-dessus sur au moins une partie dudit végétal ou du sol à proximité dudit végétal,

en particulier ladite partie dudit végétal étant une feuille, un fruit ou une graine, et
en particulier ledit végétal appartenant à la famille des *Poaceae* tels que le blé, le riz, le maïs, l'orge, le millet, le seigle, l'épeautre, l'engrain ou petit épeautre, ou le sésame.

Par « procédé pour stimuler l'assimilation des minéraux par un végétal », on entend un procédé qui une fois mis en œuvre favorise (stimule) la croissance et le développement du végétal.

Par ailleurs et selon un autre mode de réalisation, l'invention a pour objet le procédé tel que décrit ci-dessus, ladite partie dudit végétal étant une feuille, un fruit ou une graine.

Selon un autre mode de réalisation, l'invention a également pour objet le procédé tel que décrit ci-dessus, ledit végétal appartenant à la famille des *Poaceae* tels que le blé, le riz, le maïs, l'orge, le millet, le seigle, l'épeautre, l'engrain ou petit épeautre, ou le sésame. En particulier, l'invention a pour objet le procédé tel que décrit ci-dessus, ledit végétal étant choisi parmi le blé, le riz, le maïs, l'orge, le millet, le seigle, l'épeautre, l'engrain ou petit épeautre, et le sésame.

De plus, il convient de noter qu'en particulier l'invention a pour objet l'un des procédés tels que décrits ci-dessus, dans lequel ladite composition phytostimulante est épandue

- à raison de 50 à 200 L/ha, en particulier de 80 à 100 L/ha, notamment pour la pulvérisation aérienne ; ou
- à raison d'une quantité efficace de la souche isolée de *Chitinophaga pinensis* comprise de $10^{10}$ à $10^{14}$ UFC/ha, en particulier comprise de $10^{11}$ à $10^{13}$ UFC/ha, notamment pour les épandages aériens de type pulvérisation foliaire ; ou
- à raison d'une quantité efficace de la souche isolée de *Chitinophaga pinensis* comprise de $10^5$ à $10^9$ UFC/plante, en particulier comprise de $10^6$ à $10^8$ UFC/plante, notamment pour les épandages aériens de type pulvérisation foliaire.

Par « à raison de 50 à 200 L/ha », on entend que la composition phytostimulante de l'invention est épandue à une dose comprise de 50 à 200 L/ha. Cela signifie également que cette dose peut être comprise de 50 à 150 L/ha, de 100 à 200 L/ha, de 80 à 100 L/ha, de 50 à 100 L/ha voire de 100 à 150 L/ha.

Par « comprise de $10^{10}$ à $10^{14}$ UFC/ha », on entend que la souche de *Chitinophaga pinensis* est épandue à une dose comprise de $10^{10}$ à $10^{14}$ UFC/ha. Cela signifie également que cette dose peut être comprise de $10^{10}$ à $10^{13}$ UFC/ha, de $10^{10}$ à $10^{12}$ UFC/ha, de $10^{10}$ à $10^{11}$ UFC/ha, de $10^{11}$ à $10^{14}$ UFC/ha, de $10^{12}$ à $10^{14}$ UFC/ha, de $10^{13}$ à $10^{14}$ UFC/ha voire de $10^{11}$ à $10^{13}$ UFC/ha.

Par « comprise de $10^5$ à $10^9$ UFC/plante », on entend que la souche de *Chitinophaga pinensis* est épandue à une dose comprise de $10^5$ à $10^9$ UFC/plante. Cela signifie également que cette dose peut être comprise de $10^5$ à $10^8$ UFC/plante, $10^5$ à $10^7$ UFC/plante, de $10^5$ à $10^6$ UFC/plante, de $10^6$ à $10^9$ UFC/plante, de $10^7$ à $10^9$ UFC/plante, de $10^8$ à $10^9$ UFC/plante, $10^6$ à $10^8$ UFC/plante voire de $10^6$ à $10^7$ UFC/plante.

A titre d'illustration, il faut compter de $10^5$ plantes/ha (*e.g.* maïs) (100 000 plants à l'hectare) à $2.10^6$ plantes/ha (*e.g.* blé) (2 000 000 plants à l'hectare) sachant que le produit bactérien concentré (*cf.* la composition phytostimulante concentrée de l'invention - *cf. supra*) est utilisé à hauteur d'un kg/ha repris dans 50 à 100 L d'eau. Aussi et puisque la composition phytostimulante concentrée comprend jusqu'à $10^{12}$ UFC/g de *Chitinophaga pinensis,* cela signifie qu'il y a, pour un kilogramme de produit concentré, au moins $10^{15}$ UFC. Si ce kilogramme est repris dans 100 L d'eau, lesquels 100 L sont épandus sur un hectare, alors cela correspond à une concentration de *Chitinophaga pinensis* de $10^{15}$ UFC/ha soit $10^{10}$ UFC/plante s'il s'agit de maïs et $5.10^8$ UFC/plante s'il s'agit de blé. Ceci étant et comme il existe un peu de perte de produit du fait des manipulations successives et des techniques d'épandage, il est probable qu'*in fine* la quantité efficace épandue de la souche *Chitinophaga pinensis* soit moindre et correspond à celle précédemment décrite.

Selon un autre mode de réalisation, l'invention a également pour objet l'un des procédés tels que décrits ci-dessus, dans lequel

- la stimulation dudit végétal comprend la pulvérisation de ladite composition phytostimulante au moment du semis ou à la germination dudit végétal ; et/ou
- la protection dudit végétal comprend la pulvérisation de ladite composition phytostimulante au moment du semis ou à la germination dudit végétal ; et/ou
- le traitement dudit végétal comprend la pulvérisation de ladite composition phytostimulante aux cours de différents stades phénologiques de la plante.

Par ailleurs et dans la mesure où les procédés de l'invention comprennent l'utilisation de la souche isolée de *Chitinophaga pinensis* de l'invention *via* l'application de la composition phytostimulante telle que décrite ci-dessus, les caractéristiques particulières de celle-ci peuvent s'y appliquer (*cf. supra*). Autrement dit et selon un autre mode de réalisation, l'invention a pour objet l'un des procédés tels que décrits ci-dessus, dans lequel ladite souche isolée de *Chitinophaga pinensis* comprend au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence. *Etc.*

Au vu de ce qui précède, on comprend également qu'un autre aspect de l'invention concerne une nouvelle souche isolée de *Chitinophaga pinensis,* ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Par « souche isolée », on entend la culture d'un microorganisme unique qui a été isolée à partir de différents microorganismes présents sur et/ou dans un échantillon.

Selon un autre mode de réalisation, l'invention a pour objet la souche isolée telle que décrite ci-dessus, ladite souche isolée de *Chitinophaga pinensis* comprenant une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence.

Selon un autre mode de réalisation, l'invention a pour objet la souche isolée telle que décrite ci-dessus, ladite souche isolée de *Chitinophaga pinensis* comprenant au moins une enzyme dont la séquence d'acides nucléiques qui la code est choisie parmi les séquences SEQ ID NOs : 63 à 70. En particulier, l'invention a pour objet la souche isolée telle que décrite ci-dessus, ladite souche isolée de *Chitinophaga pinensis* comprenant au moins une enzyme dont la séquence d'acides nucléiques correspond à la séquence SEQ ID NO : 69, laquelle code pour l'enzyme de séquence SEQ ID NO : 77.

Selon un autre mode de réalisation, l'invention a pour objet la souche isolée telle que décrite ci-dessus, ladite souche isolée de *Chitinophaga pinensis* comprenant une enzyme dont la séquence d'acides nucléiques qui la code est choisie parmi les séquences SEQ ID NOs : 63 à 70. En particulier, l'invention a pour objet la souche isolée telle que décrite ci-dessus, ladite souche isolée de *Chitinophaga pinensis* comprenant une enzyme dont la séquence d'acides nucléiques correspond à la séquence SEQ ID NO : 69, laquelle code pour l'enzyme de séquence SEQ ID NO : 77.

Selon un autre mode de réalisation, l'invention a pour objet la souche isolée telle que décrite ci-dessus, ladite souche isolée de *Chitinophaga pinensis* comprenant au moins deux enzymes dont les séquences d'acides nucléiques qui les codent ont une identité d'au moins 84 % avec une séquence de référence choisie parmi les séquences SEQ ID NOs : 63 à 70, lesdites au moins deux enzymes conservant l'activité enzymatique de l'enzyme codée par la susdite séquence de référence.

Selon un autre mode de réalisation, l'invention a pour objet la souche isolée telle que décrite ci-dessus, ladite souche isolée de *Chitinophaga pinensis* comprenant au moins deux enzymes dont les séquences d'acides nucléiques qui les codent sont choisies parmi les séquences SEQ ID NOs: 63 à 70.

Selon un autre mode de réalisation, l'invention a pour objet la souche isolée telle que décrite ci-dessus, ladite souche isolée de *Chitinophaga pinensis* comprenant au moins trois enzymes dont les séquences d'acides nucléiques qui les codent ont une identité d'au moins 84 % avec une séquence de référence choisie parmi les séquences SEQ ID NOs : 63 à 70, lesdites au moins trois enzymes conservant l'activité enzymatique de l'enzyme codée par la susdite séquence de référence.

Selon un autre mode de réalisation, l'invention a pour objet la souche isolée telle que décrite ci-dessus, ladite souche isolée de *Chitinophaga pinensis* comprenant au moins trois enzymes dont les séquences d'acides nucléiques qui les codent sont choisies parmi les séquences SEQ ID NOs: 63 à 70.

Selon un autre mode de réalisation, l'invention a pour objet la souche isolée telle que décrite ci-dessus, ladite souche isolée de *Chitinophaga pinensis* comprenant au moins quatre enzymes dont les séquences d'acides nucléiques qui les codent ont une identité d'au moins 84 % avec une séquence de référence choisie parmi les séquences SEQ ID NOs : 63 à 70, lesdites au moins quatre enzymes conservant l'activité enzymatique de l'enzyme codée par la susdite séquence de référence.

Selon un autre mode de réalisation, l'invention a pour objet la souche isolée telle que décrite ci-dessus, ladite souche isolée de *Chitinophaga pinensis* comprenant au moins quatre enzymes dont les séquences d'acides nucléiques qui les codent sont choisies parmi les séquences SEQ ID NOs : 63 à 70.

Selon un autre mode de réalisation, l'invention a pour objet la souche isolée telle que décrite ci-dessus, ladite souche isolée de *Chitinophaga pinensis* comprenant au moins cinq enzymes dont les séquences d'acides nucléiques qui les codent ont une identité d'au moins 84 % avec une séquence de référence choisie parmi les séquences SEQ ID NOs : 63 à 70, lesdites au moins cinq enzymes conservant l'activité enzymatique de l'enzyme codée par la susdite séquence de référence.

Selon un autre mode de réalisation, l'invention a pour objet la souche isolée telle que décrite ci-dessus, ladite souche isolée de *Chitinophaga pinensis* comprenant au moins cinq enzymes dont les séquences d'acides nucléiques qui les codent sont choisies parmi les séquences SEQ ID NOs : 63 à 70.

Selon un autre mode de réalisation, l'invention a pour objet la souche isolée telle que décrite ci-dessus, ladite souche isolée de *Chitinophaga pinensis* comprenant au moins six enzymes dont les séquences d'acides nucléiques qui les codent ont une identité d'au moins 84 % avec une séquence de référence choisie parmi les séquences SEQ ID NOs : 63 à 70, lesdites au moins six enzymes conservant l'activité enzymatique de l'enzyme codée par la susdite séquence de référence.

Selon un autre mode de réalisation, l'invention a pour objet la souche isolée telle que décrite ci-dessus, ladite souche isolée de *Chitinophaga pinensis* comprenant au moins six enzymes dont les séquences d'acides nucléiques qui les codent sont choisies parmi les séquences SEQ ID NOs : 63 à 70.

Selon un autre mode de réalisation, l'invention a pour objet la souche isolée telle que décrite ci-dessus, ladite souche isolée de *Chitinophaga pinensis* comprenant au moins sept enzymes dont les séquences d'acides nucléiques qui les codent ont une identité d'au moins 84 % avec une séquence de référence choisie parmi les séquences SEQ ID NOs : 63 à 70, lesdites au moins sept enzymes conservant l'activité enzymatique de l'enzyme codée par la susdite séquence de référence.

Selon un autre mode de réalisation, l'invention a pour objet la souche isolée telle que décrite ci-dessus, ladite souche isolée de *Chitinophaga pinensis* comprenant au moins sept enzymes dont les séquences d'acides nucléiques qui les codent sont choisies parmi les séquences SEQ ID NOs : 63 à 70.

Selon un autre mode de réalisation, l'invention a pour objet la souche isolée telle que décrite ci-dessus, ladite souche isolée de *Chitinophaga pinensis* comprenant au moins huit enzymes dont les séquences d'acides nucléiques qui les codent ont une identité d'au moins 84 % avec une séquence de référence choisie parmi les séquences SEQ ID NOs : 63 à 70, lesdites au moins huit enzymes conservant l'activité enzymatique de l'enzyme codée par la susdite séquence de référence.

Selon un autre mode de réalisation, l'invention a pour objet la souche isolée telle que décrite ci-dessus, ladite souche isolée de *Chitinophaga pinensis* comprenant au moins huit enzymes dont les séquences d'acides nucléiques qui les codent correspondent aux séquences SEQ ID NOs : 63 à 70.

Selon un autre mode de réalisation, l'invention a pour objet la souche isolée telle que décrite ci-dessus, ladite souche isolée de *Chitinophaga pinensis* comprenant au moins une séquence d'acides nucléiques génomique ayant une identité d'au moins 95 % avec une séquence choisie parmi les contigs de séquences SEQ ID NOs : 22 à 61.

Selon un autre mode de réalisation, l'invention a pour objet la souche isolée telle que décrite ci-dessus, ladite souche isolée de *Chitinophaga pinensis* comprenant au moins une séquence d'acides nucléiques génomique choisie parmi les contigs de séquences SEQ ID NOs : 22 à 61.

Selon un autre mode de réalisation, l'invention a pour objet la souche isolée telle que décrite ci-dessus, ladite souche isolée de *Chitinophaga pinensis* comprenant au moins une séquence d'acides nucléiques génomique ayant une identité d'au moins 80 % avec une séquence choisie parmi les contigs de séquences SEQ ID NOs : 1 à 21.

Selon un autre mode de réalisation, l'invention a pour objet la souche isolée telle que décrite ci-dessus, ladite souche isolée de *Chitinophaga pinensis* comprenant au moins une séquence d'acides nucléiques génomique choisie parmi les contigs de séquences SEQ ID NOs : 1 à 21.

Selon un autre mode de réalisation, l'invention a pour objet la souche isolée telle que décrite ci-dessus, ladite souche isolée de *Chitinophaga pinensis* comprenant un ADN ribosomique (ADNr) 16S dont la séquence d'acides nucléiques a une identité d'au moins 99 % avec la séquence SEQ ID NO : 62.

Selon un autre mode de réalisation, l'invention a pour objet la souche isolée telle que décrite ci-dessus, ladite souche isolée de *Chitinophaga pinensis* comprenant un ADN ribosomique (ADNr) 16S dont la séquence d'acides nucléiques est la séquence SEQ ID NO : 62.

Selon un autre mode de réalisation, l'invention a pour objet la souche isolée telle que décrite ci-dessus, ladite souche isolée de *Chitinophaga pinensis* étant la souche ARDCPP14 déposée le 23 avril 2021 à la Collection Nationale de Cultures de Microorganismes (CNCM ; Institut Pasteur, 25-28 rue du Docteur Roux, F-75724 PARIS CEDEX 15) sous le numéro CNCM I-5676.

Selon un autre mode de réalisation, l'invention a pour objet la souche isolée telle que décrite ci-dessus, ladite souche isolée de *Chitinophaga pinensis* étant la souche ARDCPP14 déposée le 23 avril 2021 à la CNCM sous le numéro CNCM I-5676 et/ou un de ses mutants (fonctionnels).

Par « un de ses mutants », on entend des souches mutantes fonctionnelles obtenues par mutations ou manipulations génétiques d'une souche de référence, laquelle dans le contexte de l'invention est la souche isolée de *Chitinophaga pinensis* étant la souche ARDCPP14 déposée le 23 avril 2021 à la CNCM sous le numéro CNCM I-5676. Par « souches mutantes fonctionnelles », on entend que ces mutants conservent les mêmes propriétés physiologiques que la souche de référence (*i.e.* CNCM I-5676) voire peuvent présenter des propriétés physiologiques améliorées. Aussi, un des objets de l'invention concerne une souche isolée de *Chitinophaga pinensis,* ladite souche isolée étant une souche mutante de la souche isolée de *Chitinophaga pinensis* étant la souche ARDCPP14 déposée le 23 avril 2021 à la CNCM sous le numéro CNCM I-5676.

Par ailleurs et au vu de ce qui précède, on comprend qu'un objet de l'invention concerne la souche isolée de *Chitinophaga pinensis* telle que décrite ci-dessus, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence, ladite souche isolée de *Chitinophaga pinensis* étant en particulier la souche ARDCPP14 déposée le 23 avril 2021 à la CNCM sous le numéro CNCM I-5676.

A tout égard, il convient de noter que les différents aspects de l'invention, tout comme les différents modes de réalisation de celle-ci sont interdépendants. Ces derniers peuvent donc être combinés entre eux à foison pour obtenir des aspects et/ou des modes de réalisation préférés de l'invention non explicitement décrits. Ceci est également valable pour l'ensemble des définitions fourni dans la présente description, lequel s'applique à tous les aspects de l'invention et ses modes de réalisation.

En outre, la présente invention est illustrée, sans toutefois s'y limiter, par les Figures et Exemples suivants.

## LISTE DES FIGURES

La **figure 1** représente la production d'acide indole-3-acétique (IAA) par la souche ARDCPP14. Concentration mesurée par spectrophotométrie après ajout du réactif de Salkowski dans le surnageant de culture de la souche ARDCPP14. La concentration a été calculée après mise en place d'une gamme étalon d'IAA, puis ramenée à la matière sèche de bactérie contenue dans la culture liquide initiale.

La **figure 2** représente l'aspect visuel macroscopique de la production d'exopolysaccharide (EPS) par la souche ARDCPP14.

La **figure 3** représente l'apparition d'un précipité (indiqué par une flèche) après l'ajout d'éthanol glacial à 96 %, lequel témoigne de la production d'exopolysaccharide (EPS) par la souche ARDCPP14.

La **figure 4** représente les effets de la souche ARDCPP14 sur le poids sec des grains, du blé en condition de stress hydrique ou non. Chaque barre représente une augmentation (valeur positive) du poids sec des grains en présence de la souche ARDCPP14, comparé au témoin non inoculé dans les mêmes conditions d'arrosage ou de stress hydrique.

La **figure 5** représente le poids sec des grains du blé en condition de stress hydrique ou non, en présence de la souche ARDCPP14 ou non (NI : non inoculé), lors de deux essais différents (A (No. 3) et B (No. 4)).

La **figure 6** représente le pH, la concentration de saccharose et la densité optique à 600 nm mesurés au cours de la croissance de la souche *Chitinophaga pinensis* ARDCPP14 en fermenteur de 30 L.

**EXEMPLES**

**(I) Isolement et identification de la souche *Chitinophaga pinensis* ARDCPP14**

*Isolement bactérien*

Des échantillons de sol ont été collectés dans la rhizosphère de différentes cultures en plein champ, dans la région Champagne-Ardenne, en France. Une extraction bactérienne a été réalisée, et les souches bactériennes ont été isolées et conservées dans des cryotubes à - 80°C.

*Identification moléculaire des souches bactériennes*

Après leur isolement, les souches bactériennes isolées ont été envoyées à MicrobesNG pour séquençage. Ceci a été réalisé sur l'appareil Illumina® NovaSeq 6000 et une identification de l'ADN ribosomique (ADNr) 16S a été réalisée par une approche *in silico* à l'aide de l'outil Barrnap (BAsic Rapid Ribosomal RNA Predictor). Les séquences obtenues ont ensuite été alignées dans la base de données SINA rRNA (Quast C, Pruesse E, Yilmaz P, Gerken J, Schweer T, Yarza P, Peplies J, Glöckner FO (2013) The SILVA ribosomal RNA gene database project: improved data processing and web-based tools. Nucl. Acids Res. 41 (D1): D590-D596.) afin d'identifier les espèces bactériennes isolées.

Parmi ces souches, la souche *Chitinophaga pinensis* **ARDCPP14** a été identifiée dont le séquençage a permis de caractériser :

- les contigs de séquences SEQ ID NOs : 1 à 61 ;
- l'ADNr 16S de séquence SEQ ID NO : 62 ; et
- huit enzymes particulières de séquences SEQ ID NOs : 71 à 78 dont le cadre de lecture ouvert (ORF) comprend les séquences SEQ ID NOs : 63 à 70.

Par ailleurs, celle-ci a été déposée le 23 avril 2021 à la Collection Nationale de Cultures de Microorganismes (CNCM ; Institut Pasteur, 25-28 rue du Docteur Roux, F-75724 PARIS CEDEX 15) sous le numéro CNCM I-5676.

**(II) Production d'acide indole-3-acétique (IAA) par la souche ARDCPP14**

*Matériels & Méthodes*

Les souches bactériennes ont été inoculées à 100 $\mu$L de solution bactérienne dans un tube de culture contenant 7 mL de milieu riche TSB (Tryptic Soy Broth), supplémenté en tryptophane (Trp, 0,5 g.L-1). Après une nuit d'incubation à 110 rpm à 30°C, cinq millilitres de cette pré-culture ont été transférés dans 45 mL de TSB-Trp dans un Erlenmeyer de 500 mL. Les cultures ont été incubées à 110 rpm à 30°C pendant 72 heures, puis centrifugées pendant 10 minutes à 6000 g. Le culot a ensuite été utilisé pour obtenir la masse sèche, tandis que le surnageant a été utilisé pour l'analyse quantitative de l'IAA. La détermination de la concentration en IAA a été basée sur la méthode décrite par Gordon et Weber, 1951 (Gordon, S.A., and Weber, R.P. (1951). Colorimetric Estimation of Inodoleacetic Acid. Plant Physiol. 26, 192-195). Un millilitre du surnageant a été mélangé à deux millilitres de réactif de Salkowski, puis incubé à température ambiante pendant 25 minutes. La densité optique à 530 nm a ensuite été mesurée grâce à un spectrophotomètre. La concentration en IAA a été déterminée à l'aide d'une courbe d'étalonnage allant de 0 à 100 $\mu$g.mL$^{-1}$ et a été corrélée à la masse sèche bactérienne mesurée.

*Résultats*

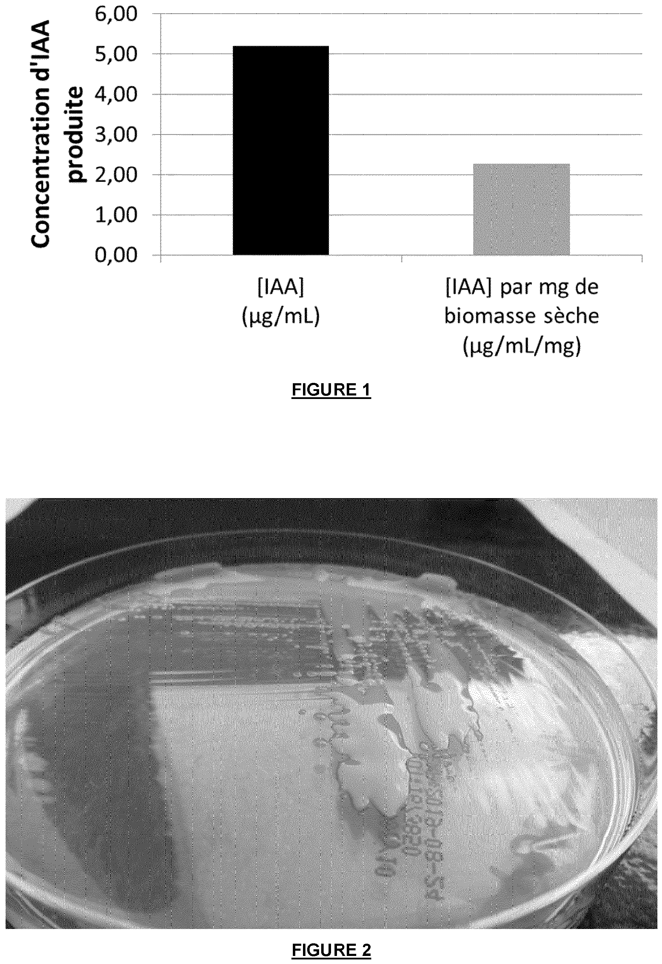

Comme illustré à la figure 1, il a été démontré que la souche ARDCPP14 est capable de produire de l'IAA : 5,20 $\mu$g.mL$^{-1}$ d'IAA ont été mesurés dans le surnageant, ce qui correspond à 2,27 $\mu$g d'IAA par mg de biomasse sèche.

**(III) Production d'exopolysaccharide (EPS) par la souche ARDCPP14**

*Matériels & Méthodes*

Les souches bactériennes ont été inoculées sur une boîte de Pétri contenant le milieu solide R2A, qui est un milieu minéral adapté aux bactéries du sol. Les plaques ont ensuite été incubées à 30°C pendant 48 heures. Une observation macroscopique permet une première détection de la production d'EPS. Les souches productrices d'EPS forment des colonies lisses, bombées et mucoïdes.

Une validation de la production d'EPS a ensuite été effectuée sur des bactéries après une croissance à 30°C pendant 45 heures :

Composition du milieu de culture :

- Saccharose 10 g/L
- Peptones de soja 10 g/L
- $KH_2PO_4$ 2,5 g/L
- NaCl 1 g/L

Conditions de culture :

- Fioles 500 mL à baffle
- Agitation : 180 rpm
- Température : 30°C
- Temps de prélèvement : 45 h

La culture bactérienne a été centrifugée à 16 000 RCF pendant 30 min et le surnageant a été récupéré. Ensuite, un volume d'éthanol glacial (conservé à -20 °C) à 96 % a été ajouté à un volume de surnageant récupéré et le mélange a été conservé au réfrigérateur 24 h, le temps de la réaction. Si un précipité blanc apparaît, cela signifie qu'un EPS produit par la souche bactérienne est présent dans le surnageant.

*Résultats*

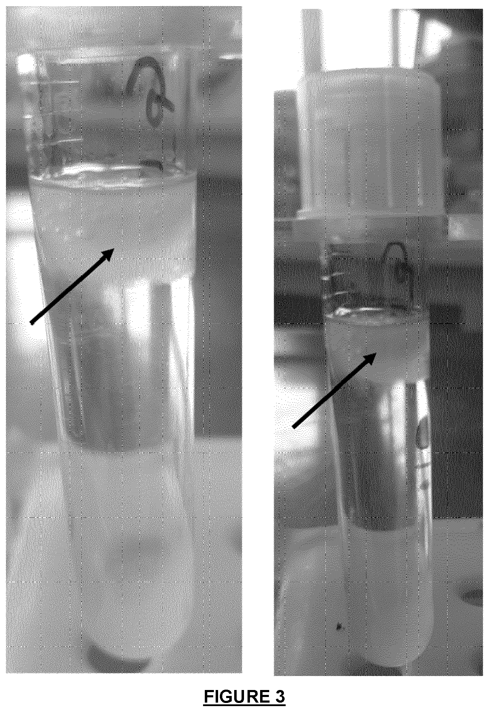

Comme illustré à la figure 2, il a été démontré que la souche ARDCPP14 est capable de produire des EPS, ce qui a d'ailleurs été confirmé par un test de précipitation dans l'éthanol glacial à 96 %. En effet, la formation d'un précipité a été observée confirmant la production d'EPS par la souche ARDCPP14 (*cf.* figure 3).

**(IV) Stress hydrique du blé - Effet de la souche ARDCPP14**

*Matériels & Méthodes*

Culture bactérienne et inoculation

Les souches bactériennes ont été inoculées à 100 $\mu$L de solution bactérienne dans un Erlenmeyer de 500 mL contenant 100 mL de milieu riche TSB (Tryptic Soy Broth). Après 48 heures d'incubation à 110 rpm à 30°C, les cultures ont été transférées dans des tubes de 50 mL et centrifugées à 10 000 rpm pendant 10 minutes. Le culot a été remis en suspension dans un volume d'eau physiologique stérile pour obtenir une concentration finale de $4.10^8$ UFC.mL$^{-1}$ (*Unit forming colony* par millitre). Chaque graine a été inoculée avec 250 $\mu$L de suspension bactérienne, soit $10^8$ UFC par graine.

Culture des plantes et conditions de stress

Des pots ont été remplis de terreau et 15 graines ont été semées à 2-3 cm de profondeur et bactérisées ($10^8$ UFC/graine). Pour chaque souche bactérienne, 8 pots ont été préparés : 4 pots bien arrosés pendant toute l'expérience et 4 pots stressés par la sécheresse. Des témoins non inoculés ont également été préparés, et les graines ont été inoculées avec 250 $\mu$L d'eau physiologique, au lieu de la solution bactérienne. Les conditions de culture étaient de 14 heures de jour (23°C) pour 10 heures de nuit (18°C). L'humidité relative était de 60 % et l'intensité lumineuse de 25 klux par jour. Les plantes ont été arrosées chaque jour par sub-irrigation. Dix jours après le semis, le nombre de plantules par pot a été réduit à 12, afin d'homogénéiser les pots. Lorsque les plantes de blé ont atteint le stade de la floraison, un stress dû à la sécheresse a été induit interrompant complètement l'arrosage pendant 10 jours.

Détermination de l'activité de promotion de la croissance des plantes par des mesures de rendement

Plusieurs paramètres ont été mesurés et calculés pour comparer les rendements des différentes conditions expérimentales. Tout d'abord, le poids des parties aériennes a été mesuré. Ensuite, la tige et l'épi ont été séparés. Les grains ont été prélevés sur les épis, séchés pendant 48 heures à 50°C, pesés et comptés. Toutes ces mesures ont été effectuées pour 2 épis pour des raisons logistiques, soit 6 mesures pour chaque pot. A partir de ces mesures, le poids de mille grains a été calculé.

Parmi ces paramètres, le poids sec des grains est important puisqu'il reflète l'amélioration de la production de la culture. Aussi et à l'échelle d'un champ, le poids sec des grains est le reflet du rendement.

Analyse statistique

Les données recueillies ont été analysées à l'aide d'une ANOVA à sens unique. Les moyennes et les erreurs standard pour les paramètres de rendement ont été calculées pour 18 valeurs répétées. Les différences significatives entre les traitements ont été analysées sur la base de l'ANOVA et du test t de Student sous un niveau de significativité de $p \leq 0,05$.

*Résultats*

Tableau 1. Effet de la souche ARDCPP14 sur le developpement du ble en condition d'arrosage normal ou en condition de stress hydrique

| Paramètres mesurés Essai No. | | Traitement | | | | | |
|---|---|---|---|---|---|---|---|
| | | Condition arrosée | | | Stress hydrique | | |
| | | Non inoculé | ARDCPP14 | Différence (%) | Non inoculé | ARDCPP14 | Différence (%) |
| **Poids plante entière** | 1 | 6,53 | **6,95** | 6,4 | 5,73 | 6,71*** | 17,1 |
| | 2 | 4,60 | **5,28*** | 14,8 | 4,09 | **4,61*** | 12,7 |
| | 3 | 5,93 | **6,47*** | 9,1 | 4,74 | **5,21*** | 9,9 |
| | 4 | 5,89 | 5,87 | -0,3 | 4,63 | **5,54*** | 19,7 |
| | 5 | 5,18 | **5,73** | 10,6 | 3,58 | **3,70** | 3,4 |
| | *Moyenne* | *5,63* | *6,06* | *8,1* | *4,55* | *5,15* | *12,5* |
| **Nombre de grains** | 1 | 91,96 | **94,04** | 2,3 | 80,54 | **90,46*** | 12,3 |
| | 2 | 70,08 | 69,92 | -0,2 | 67,92 | **69,54** | 2,4 |
| | 3 | 93,50 | **95,75** | 2,4 | 87,67 | **92,79** | 5,8 |
| | 4 | 86,83 | **88,75** | 2,2 | 77,67 | **82,04** | 5,6 |
| | 5 | 82,21 | **90,54*** | 10,1 | 58,25 | **69,00*** | 18,5 |
| | *Moyenne* | *84,92* | *87,80* | *3,4* | *74,41* | *80,77* | *8,9* |
| **Poids sec des grains** | 1 | 2,94 | **3,22** | 9,5 | 2,46 | **2,89*** | 17,5 |
| | 2 | 1,96 | **2,11** | 7,7 | 1,40 | **1,56** | 11,4 |
| | 3 | 2,75 | **2,98*** | 8,4 | 1,39 | **1,81*** | 30,2 |
| | 4 | 2,59 | **2,72** | 5,0 | 1,63 | **2,04** | 25,2 |
| | 5 | 1,98 | **2,22** | 12,1 | 0,72 | **1,05*** | 45,8 |
| | *Moyenne* | *2,44* | *2,65* | *8,5* | *1,52* | *1,87* | *26,0* |
| **Poids de mille grains** | 1 | 31,97 | **33,77**** | 5,6 | 30,52 | **31,87*** | 4,4 |
| | 2 | 27,44 | **29,78** | 8,5 | 20,12 | **22,43** | 11,5 |
| | 3 | 29,32 | **30,99**** | 5,7 | 15,88 | **20,24*** | 27,5 |
| | 4 | 30,04 | 29,90 | -0,5 | 20,30 | **24,40** | 20,2 |
| | 5 | 23,91 | **24,07** | 0,7 | 10,66 | **13,59*** | 27,5 |
| | *Moyenne* | *28,54* | *29,70* | *4,0* | *19,50* | *22,51* | *18,2* |

Les **nombres en gras** indiquent que les résultats obtenus avec la souche ARDCPP14 sont supérieurs à ceux obtenus avec le témoin non inoculé dans les mêmes conditions. Les *nombres en italique* se réfèrent à la valeur moyenne pour chacun des paramètres mesurés sur cinq essais. * $p \leq 0,05$ ; ** $p \leq 0,01$ et *** $p \leq 0,001$.

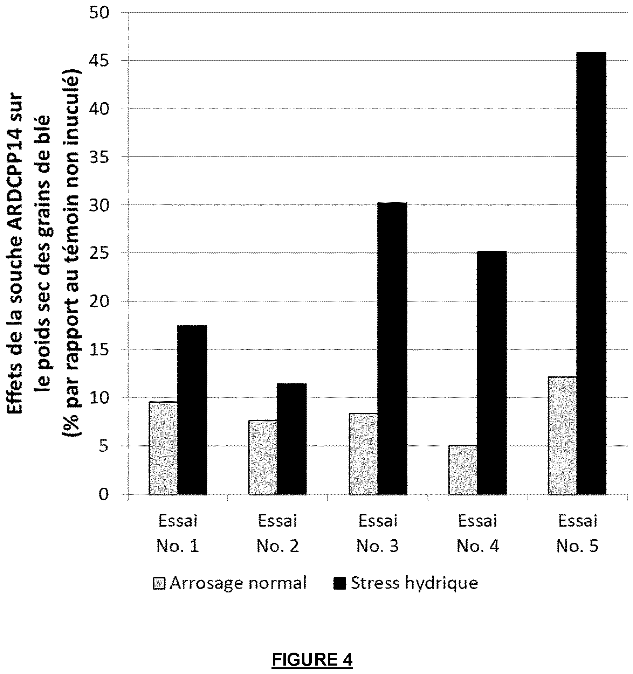

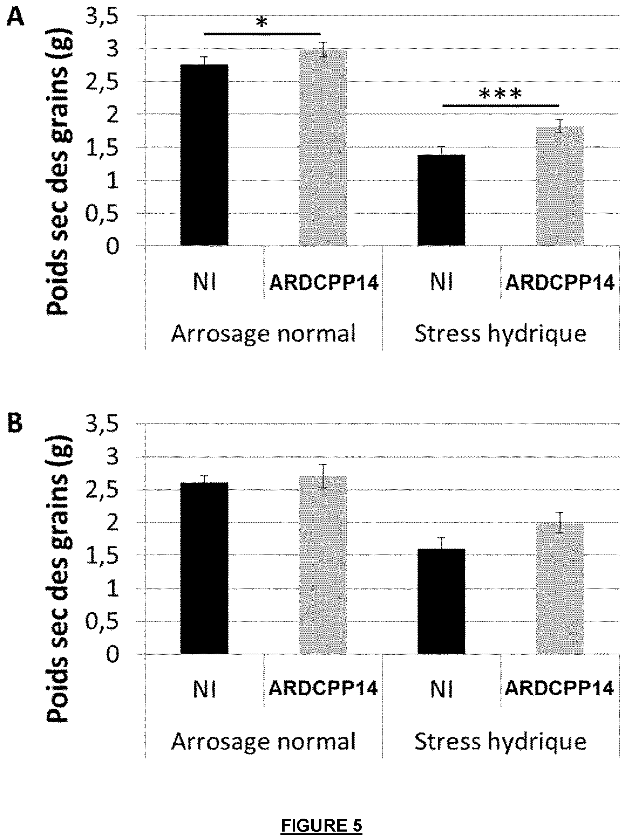

Comme illustré ci-dessus et aux figures 4 et 5, il a été démontré que la présence de la souche ARDCPP14 améliore significativement les caractéristiques du blé lorsque celui-ci est soumis à un stress hydrique. Ceci est également vrai lorsque le blé a été arrosé normalement et ne souffre d'aucun stress hydrique.

Par ailleurs, il convient de noter que des résultats similaires sont obtenus lorsque le végétal traité est l'orge.

En définitive, la souche ARDCPP14 a démontré un rôle dans la biostimulation du blé et de l'orge, d'une part en tant qu'agent de biofertilisation (*i.e.* amélioration du poids sec des grains (= rendement) en condition normal) et d'autre part en tant qu'agent de lutte contre le stress hydrique (*i.e.* amélioration du poids sec des grains (= rendement) en condition de stress hydrique).

### (V) Composition phytostimulante d'enrobage

*Matériels & Méthodes*

Moût de bactéries

Les moûts de bactéries ont été fournis par le service Biotechnologie d'ARD.

La souche sélectionnée a été la souche *Chitinophaga pinensis* ARDCPP14. La croissance de cette souche a été réalisée dans un milieu semi-défini, avec du saccharose comme source principale de carbone. La récolte a été réalisée après un temps de culture variant de 48 à 72 heures.

En détails :

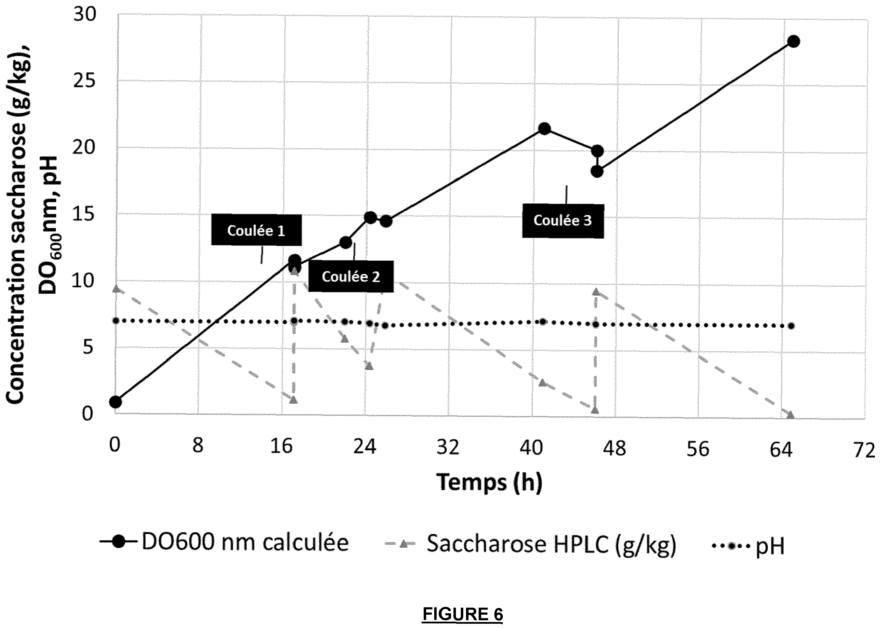

Une pré-culture a été réalisée dans les conditions présentées au tableau 2, puis une culture en continu-discontinu, dite de type *fed-batch*, a été réalisée dans les conditions présentées au tableau 3 et en fermenteur de 30 L. Au cours de cette culture en fermenteur, ont été mesurés le pH, la concentration de saccharose et la densité optique à 600 nm, laquelle témoigne de la croissance de la souche *Chitinophaga pinensis* ARDCPP14 (*cf.* figure 6).

**Tableau 2. Conditions de culture de la pré-culture en erlenmeyer de 500 mL**

| Pré-culture : Erlens 500 mL | |
|---|---|
| Cuverie | Fioles 500 mL 1 baffle |
| Milieu de culture | Saccharose 15 g/L, Peptones de soja 10 g/L, $KH_2PO_4$ 2,5 g/L, NaCl 1 g/L, $MgSO_4$ 1 g/L |
| Volume de milieu | 100 mL (x 3 fioles) |
| Température | 30°C |
| pH initial | 7,5 |
| Agitation | 180 rpm sur table d'agitation orbitale 25 mm |
| Quantité d'inoculum | 800 $\mu$L |
| Durée | 20h-24h |

**Tableau 3. Conditions de culture en fermenteur de 30 L**

| Culture principale : Fermenteur 30 L - *Fed-Batch* | |
|---|---|
| Cuverie | Fermenteur 30 L Sartorius Biostat® Cplus |
| Milieu de culture batch | Saccharose 10 g/L, Peptones de soja 5 g/L, $(NH_4)_2SO_4$ 2,0 g/L, $KH_2PO_4$ 2,5 g/L, NaCl 1 g/L, $MgSO_4$ 1 g/L, solution éléments traces 0,1 % (v/v) |
| Milieu *fed-batch* | Milieu batch concentré15 fois |
| Type de *fed-batch* | Coulées |
| Volume de milieu | 15 L batch + 5 L fed-batch |
| Température | 30°C |
| pH | 7 ; régulation avec NaOH 15 % + $H_2SO_4$ 10 % |
| $pO_2$ | maintien >30 % |

(suite)

| Culture principale : Fermenteur 30 L - *Fed-Batch* | |
|---|---|
| Cascade pO$_2$ | augmentation agitation puis aération |
| Agitation | 400 - 800 rpm (1 hélice Rushton et 2 hélices tripales) |
| Pression de couverture | 1 bar |
| Aération | 0,5 vvm (7,5 L/min) à 1 vvm (15 L/min) |
| Taux d'inoculation | 300 mL (2 % v/v) |
| Durée batch | 24h |
| Durée fed-batch | 24h-48h |
| Coulée | Cible saccharose 10-15 g/kg |

Centrifugation : obtention d'une crème de bactéries

Les moûts de bactéries ont été séparés par centrifugation à 4°C. La centrifugeuse utilisée a été une centrifugeuse batch Thermofisher® RC 12BP+. Le temps de centrifugation des moûts a été fixé à 1 heure et la vitesse d'accélération à 6 000 G.

A l'issu de cette étape, un culot bactérien a été obtenu, lequel a été appelé « crème de bactéries ».

Préparation de la composition phytostimulante d'enrobage

Les bactéries étant très sensibles au séchage, différents co-formulants (dont des agents protecteurs) ont été ajoutés à la crème de bactéries afin de maintenir une meilleure viabilité des bactéries au cours du séchage et du stockage. Il s'agit du glycérol, de la gomme arabique et du polyvinylpyrrolidone (PVP). A également été ajouté de l'acide ascorbique pour son rôle antioxydant au niveau des membranes et protecteur de l'intégrité membranaire.

Afin de faciliter le mélange des co-formulants à la crème de bactéries, ces co-formulants ont été préalablement mis en solution dans le milieu nutritif utilisé pour la production de la souche *Chitinophaga pinensis* ARDCPP14 pour garantir le maximum de viabilité au stockage et à l'utilisation.

La solution de co-formulant a été stérilisée à l'autoclave (121°C / 20 min) avant utilisation, et ont été ajoutés du lait écrémé et de l'acide ascorbique pour éviter leur dégradation. Cette solution a ensuite été ajoutée à la crème de bactéries. Pour réaliser ce mélange « crème de bactéries / solution de co-formulant », la crème de bactéries issue de la centrifugation a été placée dans un flacon en verre et additionnée de la solution de co-formulant à 50/50 (m/m) puis agitée sur une plaque d'agitation à 20°C pendant 30 minutes.

La liste des co-formulants utilisés et leurs doses sont présentées dans le tableau 4 ci-après.

**Tableau 4. Liste des co-formulants utilisés et leurs doses**

| Additif | Solution de co-formulant (concentration en % m/m) | Crème de bactéries avec co-formulants (concentration en % m/m) |
|---|---|---|
| Glycérol | 4 | 2 |
| PVP | 4 | 2 |
| Gomme arabique | 10 | 5 |
| Lait écrémé | 6 | 3 |
| Acide ascorbique | 0,04 | 0,02 |

**(VI) Enrobage des graines - enrobage par pulvérisation sur lit fluidisé**

La fluidisation consiste à faire passer de l'air à travers un lit de particules, supportés par une grille, pour les mettre en suspension. L'air assure un transfert de chaleur à la surface des particules et un mouvement cyclique des particules de la zone de pulvérisation à la zone de séchage. L'efficacité de transfert d'énergie est liée à la hauteur du lit de particules (dépendant de la quantité et de la densité du support), du débit d'air de fluidisation et de l'humidité de l'air de fluidisation.

Ce type de technologie permet de faire du séchage, de l'enrobage, de la granulation ou encore de l'encapsulation.

Pour exemple et pout le lit fluidisé peut être utilisé l'appareil Glatt GPCG3.

*Matériels & Méthodes*

Le procédé d'enrobage par fluidisation permet de réaliser un enrobage en continu des particules. Ce procédé a été réalisé en trois étapes :

- Fluidisation du support (graines = semences) ;
- Pulvérisation de la matière active (bactéries sous forme de crème additionnée de co-formulants = composition phytostimulante) enrobant le support ; et
- Séchage et filmification de l'enrobage.

Le matériau enrobant (*i.e.* composition phytostimulante) sous la forme liquide a été pulvérisé sur le support (graines) au moyen d'une buse de pulvérisation. La buse bifluide a assuré le passage de la composition phytostimulante (*i.e.* crème additionnée de co-formulants) apportée par une pompe et l'a dispersée en fines gouttelettes grâce à un flux d'air comprimé. Les gouttelettes sont entrées en contact de la surface du support (*i.e.* graines) et s'y sont déposées en formant un film d'enrobage à la température de séchage choisie.

Tout au long du procédé d'enrobage, les paramètres tels que le débit d'air de fluidisation, la température de l'air en entrée et en sortie et le débit d'incorporation de la composition phytostimulante ont été suivis et modifiés en fonction du comportement des graines enrobées en fluidisation (*cf.* tableau 5).

**Tableau 5. Paramètres de fluidisation et de pulvérisation retenus**

| Paramètres | Consignes |
|---|---|
| Quantité de graines traitée par batch (kg) | 1,5 |
| Température en entrée d'air (°C) | 40 |
| Température produit (°C) | 45 |
| Débit d'air au départ (m$^3$/h) | 130 |
| Débit d'air pendant l'enrobage (m$^3$/h) | De 140 à 160 |
| Débit d'air pendant le séchage (m$^3$/h) | De 140 à 160 |
| Débit de pulvérisation (kg/h) | 0,8 |
| Pression de pulvérisation (bar) | 1,5 |

Pour 1,5 kg de graines, 300 g de composition phytostimulante ont été pulvérisés à un débit de 0,3 kg/h sur les graines, puis une phase de séchage à 30°C pendant une durée de 10 minutes a été opérée pour obtenir une matière sèche ≥ 95 %.

**Revendications**

1. Utilisation d'une souche isolée de *Chitinophaga pinensis* en tant qu'agent de biostimulation, en particulier en tant qu'agent de lutte contre le stress hydrique et/ou en tant qu'agent biofertilisant.

2. Utilisation d'une souche isolée de *Chitinophaga pinensis* selon la revendication 1 dans la prévention et/ou le traitement du stress hydrique subi par un végétal,
en particulier ledit végétal appartenant à la famille des *Poaceae* tels que le blé, le riz, le maïs, l'orge, le millet, le seigle, l'épeautre, l'engrain ou petit épeautre, ou le sésame.

3. Utilisation d'une souche isolée de *Chitinophaga pinensis* selon la revendication 1 pour stimuler l'assimilation des minéraux par un végétal,
en particulier ledit végétal appartenant à la famille des *Poaceae* tels que le blé, le riz, le maïs, l'orge, le millet, le seigle, l'épeautre, l'engrain ou petit épeautre, ou le sésame.

**4.** Utilisation selon l'une quelconque des revendications 1 à 3, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence,
en particulier, ladite souche isolée de *Chitinophaga pinensis* étant la souche ARDCPP14 déposée le 23 avril 2021 à la CNCM sous le numéro CNCM I-5676.

**5.** Composition phytostimulante comprenant :

- une souche isolée de *Chitinophaga pinensis* en tant que principe actif ; ou
- une souche isolée de *Chitinophaga pinensis* en tant que premier principe actif en association avec au moins un autre principe actif choisi parmi :

  ◦ un autre agent de biostimulation ;
  ◦ un produit phytopharmaceutique de biocontrôle ;
  ◦ un engrais ; et
  ◦ leurs mélanges,

    en particulier, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence,
    en particulier, ladite souche isolée de *Chitinophaga pinensis* étant la souche ARDCPP14 déposée le 23 avril 2021 à la CNCM sous le numéro CNCM I-5676.

**6.** Composition phytostimulante selon la revendication 5, comprenant une quantité efficace de la souche isolée de *Chitinophaga pinensis* comprise de $10^6$ à $10^{12}$ UFC/g, plus particulièrement comprise de $10^8$ à $10^{10}$ UFC/g, ladite composition phytostimulante étant éventuellement obtenue après une étape de préparation la rendant prête à l'épandage.

**7.** Composition phytostimulante d'enrobage comprenant une souche isolée de *Chitinophaga pinensis* et éventuellement au moins un agent de fixation,

    en particulier, ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence,
    en particulier, ladite souche isolée de *Chitinophaga pinensis* étant la souche ARDCPP14 déposée le 23 avril 2021 à la CNCM sous le numéro CNCM I-5676.

**8.** Semence enrobée comprenant une graine de végétal, ladite graine de végétal étant enrobée par la composition phytostimulante d'enrobage selon la revendication 7,
en particulier ladite graine de végétal appartenant à un végétal de la famille des *Poaceae* tels que le blé, le riz, le maïs, l'orge, le millet, le seigle, l'épeautre, l'engrain ou petit épeautre, ou le sésame.

**9.** Procédé de protection et/ou de traitement du stress hydrique subi par un végétal comprenant l'application de la composition phytostimulante selon la revendication 5 ou 6 sur au moins une partie dudit végétal ou du sol à proximité dudit végétal,

    en particulier ladite partie dudit végétal étant une feuille, un fruit ou une graine, et
    en particulier ledit végétal appartenant à la famille des *Poaceae* tels que le blé, le riz, le maïs, l'orge, le millet, le seigle, l'épeautre, l'engrain ou petit épeautre, ou le sésame.

**10.** Procédé pour stimuler l'assimilation des minéraux par un végétal comprenant l'application de la composition phytostimulante selon la revendication 5 ou 6 sur au moins une partie dudit végétal ou du sol à proximité dudit végétal,

    en particulier ladite partie dudit végétal étant une feuille, un fruit ou une graine, et
    en particulier ledit végétal appartenant à la famille des *Poaceae* tels que le blé, le riz, le maïs, l'orge, le millet, le seigle, l'épeautre, l'engrain ou petit épeautre, ou le sésame.

**11.** Procédé selon la revendication 9 ou 10, dans lequel ladite composition phytostimulante est épandue

- à raison de 50 à 200 L/ha, en particulier de 80 à 100 L/ha, notamment pour la pulvérisation aérienne ; ou
- à raison d'une quantité efficace de la souche isolée de *Chitinophaga pinensis* comprise de $10^{10}$ à $10^{14}$ UFC/ha, en particulier comprise de $10^{11}$ à $10^{13}$ UFC/ha, notamment pour les épandages aériens de type pulvérisation foliaire ; ou
- à raison d'une quantité efficace de la souche isolée de *Chitinophaga pinensis* comprise de $10^5$ à $10^9$ UFC/plante, en particulier comprise de $10^6$ à $10^8$ UFC/plante, notamment pour les épandages aériens de type pulvérisation foliaire.

**12.** Procédé selon l'une quelconque des revendications 9 à 11, dans lequel

- la stimulation dudit végétal comprend la pulvérisation de ladite composition phytostimulante au moment du semis ou à la germination dudit végétal ; et/ou
- la protection dudit végétal comprend la pulvérisation de ladite composition phytostimulante au moment du semis ou à la germination dudit végétal ; et/ou
- le traitement dudit végétal comprend la pulvérisation de ladite composition phytostimulante aux cours de différents stades phénologiques de la plante.

**13.** Souche isolée de *Chitinophaga pinensis,* ladite souche isolée de *Chitinophaga pinensis* comprenant au moins une enzyme dont la séquence d'acides nucléiques qui la code a une identité d'au moins 84 % avec une séquence de référence choisie parmi les séquences SEQ ID NOs : 63 à 70, ladite au moins une enzyme conservant l'activité enzymatique de l'enzyme codée par la susdite séquence de référence,
ladite souche isolée de *Chitinophaga pinensis* étant en particulier la souche ARDCPP14 déposée le 23 avril 2021 à la CNCM sous le numéro CNCM I-5676.

FIGURE 1

FIGURE 2

FIGURE 3

**FIGURE 4**

FIGURE 5

**FIGURE 6**

Europäisches
Patentamt

European
Patent Office

Office européen
des brevets

**RAPPORT DE RECHERCHE EUROPEENNE**

Numéro de la demande

**EP 22 19 2942**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2016/130586 A2 (BIOCONSORTIA INC [US]) 18 août 2016 (2016-08-18) * abrégé * * revendications 1, 9, 11-13 * * alinéas [0018], [0045], [0046] * ----- | 1-3,5-12 | INV. C12N1/20 A01N63/20 A01P21/00 ADD. C12R1/01 |
| A | GLAVINA DEL RIO TIJANA ET AL: "Complete genome sequence of Chitinophaga pinensis type strain (UQM 2034T)", STANDARDS IN GENOMIC SCIENCES, vol. 2, no. 1, 28 février 2010 (2010-02-28), pages 87-95, XP055915523, DOI: 10.4056/sigs.661199 * le document en entier * ----- | 1-13 | |
| A | PANDYA M ET AL: "Exploring plant growth promoting potential of non rhizobial root nodules endophytes of Vigna radiata", MICROBIOLOGY, CONSULTANTS BUREAU, NEW YORK, NY, US, vol. 84, no. 1, 4 février 2015 (2015-02-04), pages 80-89, XP035440945, ISSN: 0026-2617, DOI: 10.1134/S0026261715010105 [extrait le 2015-02-04] * abrégé * * table * * figures 1,2 * ----- | 1-13 | |

DOMAINES TECHNIQUES
RECHERCHES (IPC)

C12N
C12R

-/--

Le présent rapport a été établi pour toutes les revendications

| Lieu de la recherche | Date d'achèvement de la recherche | Examinateur |
|---|---|---|
| La Haye | 17 janvier 2023 | Sonnerat, Isabelle |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........................................................
& : membre de la même famille, document correspondant

EPO FORM 1503 03.82 (P04C02)

EP 4 141 103 A1

<table>
<tr><td colspan="2"></td><td>Europäisches<br>Patentamt<br>European<br>Patent Office<br>Office européen<br>des brevets</td><td>RAPPORT DE RECHERCHE EUROPEENNE</td><td>Numéro de la demande<br><br>EP 22 19 2942</td></tr>
</table>

## DOCUMENTS CONSIDERES COMME PERTINENTS

| Catégorie | Citation du document avec indication, en cas de besoin,<br>des parties pertinentes | Revendication<br>concernée | CLASSEMENT DE LA<br>DEMANDE (IPC) |
|---|---|---|---|
| A | CHIMWAMUROMBE PERCY MARUWA ET AL: "Isolation and characterization of culturable seed-associated bacterial endophytes from gnotobiotically grown Marama bean seedlings", FEMS MICROBIOLOGY ECOLOGY , vol. 92, no. 6 1 juin 2016 (2016-06-01), page fiw083, XP055915741, DOI: 10.1093/femsec/fiw083 Extrait de l'Internet: URL:https://watermark.silverchair.com/fiw0 83.pdf?token=AQECAHi208BE49Ooan9kkhW_Ercy7 Dm3ZL_9Cf3qfKAc485ysgAAAt4wggLaBgkqhkiG9w0 BBwaggqLLMIICxwIBADCCAsAGCSqGSIb3DQEHATAeB glghkgBZQMEAS4wEQQMKKHEmGcJYiF-JWAWAgEQgII CkUSpotSjtXYzEdwIcz7SbzFvG5vhiMIQ-P6WofwNT 0CZOy-SQbrxtiAMreGcu5dEA7BQ_91L9g3Sm-tEegt Y8wy1LzxHr<br>----- | 1-13 | |
| X,P | CHAI YEN NING ET AL: "Assessment of Bacterial Inoculant Delivery Methods for Cereal Crops", FRONTIERS IN MICROBIOLOGY , vol. 13 26 janvier 2022 (2022-01-26), pages 10-3389, XP055915525, DOI: 10.3389/fmicb.2022.791110 Extrait de l'Internet: URL:https://www.ncbi.nlm.nih.gov/pmc/artic les/PMC8826558/pdf/fmicb-13-791110.pdf * le document en entier *<br>----- | 1,3,5-8,<br>10 | DOMAINES TECHNIQUES<br>RECHERCHES (IPC) |

Le présent rapport a été établi pour toutes les revendications

| Lieu de la recherche | Date d'achèvement de la recherche | Examinateur |
|---|---|---|
| La Haye | 17 janvier 2023 | Sonnerat, Isabelle |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

33

## ANNEXE AU RAPPORT DE RECHERCHE EUROPEENNE
## RELATIF A LA DEMANDE DE BREVET EUROPEEN NO.

**EP 22 19 2942**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**17-01-2023**

| Document brevet cité au rapport de recherche | Date de publication | Membre(s) de la famille de brevet(s) | Date de publication |
|---|---|---|---|
| WO 2016130586 A2 | 18-08-2016 | AU 2016219488 A1 | 14-09-2017 |
| | | AU 2020286338 A1 | 28-01-2021 |
| | | AU 2021203932 A1 | 08-07-2021 |
| | | BR 112017017003 A2 | 26-06-2018 |
| | | CA 2976045 A1 | 18-08-2016 |
| | | CN 108430222 A | 21-08-2018 |
| | | CN 114698651 A | 05-07-2022 |
| | | EP 3261445 A2 | 03-01-2018 |
| | | IL 281528 A | 31-05-2021 |
| | | JP 2018507708 A | 22-03-2018 |
| | | JP 2021090464 A | 17-06-2021 |
| | | US 2018020671 A1 | 25-01-2018 |
| | | US 2020229443 A1 | 23-07-2020 |
| | | WO 2016130586 A2 | 18-08-2016 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **CAMAILLE M. ; FABRE N. ; CLÉMENT C. ; AIT BARKA E.** Plant Growth Promoting Rhizobacteria: a sustainable wat to trigger drought tolerance in wheat. *Microorganisms,* 2021, vol. 9, x
- Bacillus subtilis-Mediated Abiotic Stress Tolerance in Plants. **LASTOCHKINA O.** Bacilli and Agrobiotechnology: Phytostimulation and Biocontrol. Bacilli in Climate Resilient Agriculture and Bioprospecting. Springer, 2019
- **ZHOU GC ; WANG Y ; ZHAI S ; GE F ; LIU ZH ; DAI YJ ; YUAN S ; HOU JY.** Biodegradation of the neonicotinoid insecticide thiamethoxam by the nitrogen-fixing and plant-growth-promoting rhizobacterium Ensifer adhaerens strain TMX-23. *Appl Microbiol Biotechnol.,* Mai 2013, vol. 97 (9), 4065-74
- [52] L-tryptophan 2,3-dioxygenase (tryptophan pyrrolase) (pseudomonas fluorescens). **YUZURU ISHIMURA.** Methods in Enzymology. Academic Press, 1970, vol. 17, 429-434
- **BULLER, ANDREW R et al.** Directed evolution of the tryptophan synthase bêta-subunit for stand-alone function recapitulates allosteric activation. *Proc Natl Acad Sci U S A.,* 24 Novembre 2015, vol. 112 (47), 14599-14604
- **R.D. MOSTELLER.** Improved method for measuring the catalytic activity of tryptophan synthase $\alpha$ subunit in cell extracts. *Biochimie,* 1990, vol. 72 (12), 881-884
- **SÖDERHOLM, ANNIKA et al.** Structure and kinetics of indole-3-glycerol phosphate synthase from Pseudomonas aeruginosa: Decarboxylation is not essential for indole formation. *The Journal of biological chemistry,* 2020, vol. 295 (47), 15948-15956
- Methods of Enzymatic Analysis. Academic Press, 1974, 798-801
- **KÖRNER C ; LEHLE L ; VON FIGURA K.** Abnormal synthesis of mannose 1-phosphate derived carbohydrates in carbohydrate-deficient glycoprotein syndrome type I fibroblasts with phosphomannomutase deficiency. *Glycobiology,* Février 1998, vol. 8 (2), 165-71
- **OKAZAKI R ; OKAZAKI T ; STROMINGER JL ; MICHELSON AM.** Thymidine diphosphate 4-keto-6-deoxy-d-glucose, an intermediate in thymidine diphosphate L-rhamnose synthesis in Escherichia coli strains. *J Biol Chem.,* Octobre 1962, vol. 237, 3014-26
- **SHINABARGER D ; BERRY A ; MAY TB ; ROTHMEL R ; FIALHO A ; CHAKRABARTY AM.** Purification and characterization of phosphomannose isomerase-guanosine diphospho-D-mannose pyrophosphorylase. A bifunctional enzyme in the alginate biosynthetic pathway of Pseudomonas aeruginosa. *J Biol Chem.,* 05 Février 1991, vol. 266 (4), 2080-8
- **CAMPANELLA, J. J. ; BITINCKA, L. ; SMALLEY, J.** MatGAT: an application that generates similarity/identity matrices using protein or DNA sequences. *BMC Bioinformatics,* 2003, vol. 4, 29
- **QUAST C ; PRUESSE E ; YILMAZ P ; GERKEN J ; SCHWEER T ; YARZA P ; PEPLIES J ; GLÖCKNER FO.** The SILVA ribosomal RNA gene database project: improved data processing and web-based tools. *Nucl. Acids Res,* 2013, vol. 41 (D1), D590-D596
- **GORDON, S.A. ; WEBER, R.P.** Colorimetric Estimation of Inodoleacetic Acid. *Plant Physiol.,* 1951, vol. 26, 192-195